(19) Europäisches Patentamt
European Patent Office
Office européen des brevets

(11) EP 1 716 113 B1

(12) **EUROPEAN PATENT SPECIFICATION**

(45) Date of publication and mention of the grant of the patent:
**25.02.2009 Bulletin 2009/09**

(21) Application number: **04806216.0**

(22) Date of filing: **22.12.2004**

(51) Int Cl.:
***C07C 405/00*** (2006.01) ***C07C 59/90*** (2006.01)
***A61K 31/5575*** (2006.01) ***A61K 31/192*** (2006.01)
***A61P 37/00*** (2006.01)

(86) International application number:
**PCT/GB2004/005421**

(87) International publication number:
**WO 2005/061449 (07.07.2005 Gazette 2005/27)**

(54) **EP2- RECEPTOR AGONISTS**

EP2-REZEPTORAGONISTEN

AGONISTES DU RECEPTEUR -EP 2

(84) Designated Contracting States:
**AT BE BG CH CY CZ DE DK EE ES FI FR GB GR HU IE IS IT LI LT LU MC NL PL PT RO SE SI SK TR**

(30) Priority: **22.12.2003 GB 0329620**
**24.12.2003 US 531979 P**
**27.10.2004 US 622012 P**

(43) Date of publication of application:
**02.11.2006 Bulletin 2006/44**

(73) Proprietor: **ASTERAND UK LIMITED**
**Royston**
**Hertfordshire SG8 5HD (GB)**

(72) Inventors:
- **BORMAN, Richard Anthony, Pharmagene Lab. Ltd. Royston, Hertfordshire SG8 5HD (GB)**
- **COLEMAN, Robert Alexander, Pharmagene Lab. Ltd. Royston, Hertfordshire SG8 5HD (GB)**
- **CLARK, Kenneth Lyle, Pharmagene Lab. Ltd. Royston, Hertfordshire SG8 5HD (GB)**
- **MILLS, Keith, Pharmagene Lab. Limited Royston, Hertfordshire SG8 5HD (GB)**
- **OXFORD, Alexander William, Pharmagene Lab. Ltd. Royston, Hertfordshire SG8 5HD (GB)**
- **ZHANG, Jian, Pharmagene Lab. Ltd Royston, Hertfordshire SG8 5HD (GB)**
- **DUFF, Peter Thomas, Target Molecules Limited Southampton, Hampshire SO17 3RY (GB)**

(74) Representative: **Watson, Robert James et al**
**Mewburn Ellis LLP**
**33 Gutter Lane**
**London**
**EC2V 8AS (GB)**

(56) References cited:
**WO-A-03/037433**

- **SZCZEPAN JOZEFOWSKI ET AL.: "Exogenous but not endogenous prostanoids regulate cytokine secretion from murine bone marrow dendritic cells: EP2, DP, and IP but not EP1, EP3, and FP prostanoid receptors are involved" INTERNATIONAL IMMUNOPHARMACOLOGY, vol. 3, 1 June 2003 (2003-06-01), pages 865-878, XP002325093**
- **NIALS A T ET AL: "AH13205, A SELECTIVE PROSTANOID EP2-RECEPTOR AGONIST" CARDIOVASCULAR DRUG REVIEWS, NEVA PRESS, BRANFORD, CT, US, vol. 11, no. 2, 1993, pages 165-179, XP009004866 ISSN: 0897-5957**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- VANCHERI C ET AL: "The lung as a privileged site for the beneficial actions of PGE2" TRENDS IN IMMUNOLOGY, ELSEVIER, CAMBRIDGE, GB, vol. 25, no. 1, January 2004 (2004-01), pages 40-46, XP004481206 ISSN: 1471-4906
- KANDA N ET AL: "Prostaglandin E2 suppresses CCL27 production through EP2 and EP3 receptors in human keratinocytes" JOURNAL OF ALLERGY AND CLINICAL IMMUNOLOGY, MOSBY - YEARLY BOOK, INC, US, vol. 114, no. 6, December 2004 (2004-12), pages 1403-1409, XP004666387 ISSN: 0091-6749
- HILLOCK C J ET AL: "INHIBITORY PROSTANOID EP RECEPTORS IN HUMAN NON-PREGNANT MYOMETRIUM" EUROPEAN JOURNAL OF PHARMACOLOGY, AMSTERDAM, NL, vol. 378, no. 1, 28 July 1999 (1999-07-28), pages 99-108, XP001124311 ISSN: 0014-2999 cited in the application
- M J T ROBINSON: "Organic Stereochemistry" 2000, OXFORD UNIVERSITY PRESS , NEW YORK * pages 61-63 *
- A SALIM, R EMERSON: "Targeting interleukin-2 as treatment of psoriasis" CURRENT OPINION IN INVESTIGSTION DRUGS, vol. 2, no. 11, 2001, pages 1546-1548,
- E ANASTASSIOU ET AL.: "Prostaglandine E2 and other cyclic AMP-elevating agents modulate IL-2 and IL-2Ralpha gene expression at multiple levels" THE JOURNAL OF IMMUNOLOGY, vol. 148, no. 9, 1992, pages 2845-2852,
- F G M SNIJDEWINT ET AL.: "Prostaglandine E2 differentially modulates cytokine secretion profiles of human T-helper lymphocytes" THE JOURNAL OF IMMUNOLOGY, vol. 150, no. 12, 1993, pages 5321-5329,

## Description

This invention relates to certain stereoisomers of AH13205, (±)- trans-2-[4-(1-hydroxyhexyl)phenyl]-5-oxo-cyclopentaneheptanoic acid and their use as $EP_2$ receptor agonists. The invention also relates to pharmaceutical compositions comprising these stereoisomers, and the use of these stereoisomers and compositions to treat various diseases.

*Background to the invention*

Prostanoids comprise prostaglandins (PGs) and thromboxanes (Txs) and their receptors fall into five different classes (DP, EP, FP, IP and TP) based on their sensitivity to the five naturally occurring prostanoids, $PGD_2$, $PGE_2$, $PGF_{2\alpha}$, $PGI_2$ and $TxA_2$, respectively (Coleman, R.A., Prostanoid Receptors. IUPHAR compendium of receptor characterisation and classification, 2nd edition, 338-353, ISBN 0-9533510-3-3, 2000). EP receptors (for which the endogenous ligand is $PGE_2$) have been subdivided into four types termed $EP_1$, $EP_2$, $EP_3$ and $EP_4$. These four types of EP receptors have been cloned and are distinct at both a molecular and pharmacological level (Coleman, R.A., 2000)

$EP_2$ agonists have been shown to be effective in the treatment of a number of conditions, including (but not limited to) dysmenorrhoea (WO 03/037433), pre-term labour (GB 2 293 101), glaucoma (WO 03/040126), ocular hypertension (WO 03/040126), immune disorders (WO 03/037433), osteoporosis (WO 98/27976, WO 01/46140), asthma (WO 03/037433), allergy (WO 03/037433), bone disease (WO 02/24647), fracture repair (WO 98/27976, WO 02/24647), fertility (Breyer, R.M., et al., Ann. N.Y. Acad. Sci., 905, 221-231 (2000)), male sexual dysfunction (WO 00/40248), female sexual dysfunction (US 6,562,868), periodontal disease (WO 00/31084), gastric ulcer (US 5,576,347) and renal disease (WO 98/34916).

One known $EP_2$ agonist with good selectivity is butaprost:

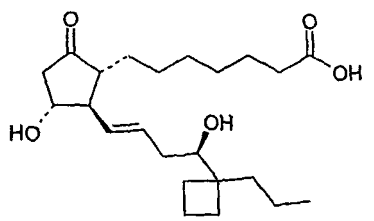

AH13205, (±)- *trans*-2-[4-(1-hydroxyhexyl)phenyl]-5-oxo-cyclopentaneheptanoic acid, is known as an $EP_2$ agonist (for example, see Hillock, C.J. and Crankshaw, D.J., European Journal of Pharmacology, 378, 99-108 (1999)).

It can also be called 7-{**2**-[*4*-(1-hydroxy-hexyl)-phenyl]-5-oxo-cyclopentyl}-heptanoic acid (fonts added for identification), and has the following structure:

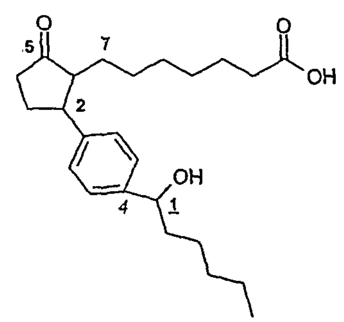

This structure has three chiral carbon atoms and hence eight possible stereoisomers. When the groups on the cyclic pentanone are in a *trans* relationship, this gives rise to four stereoisomers which are the major ones and when the groups are in a *cis* relationship, gives rise to four minor stereoisomers.

The four major stereoisomers have the following structures:

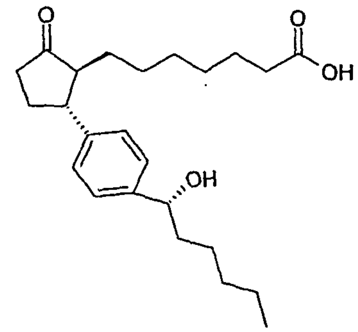

(1S,2R)-2-[4-(1-(R)-hydroxyhexyl)phenyl]-5-oxo-cyclopentaneheptanoic acid [SRR]

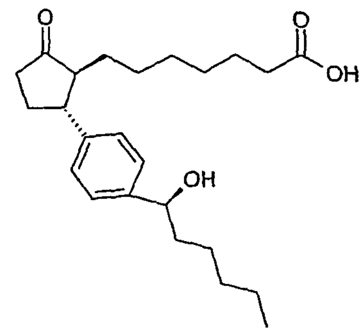

(1S,2R)-2-[4-(1-(S)-hydroxyhexyl)phenyl]-5-oxo-cyclopentaneheptanoic acid [SRS]

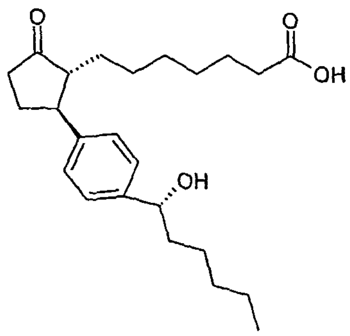

(1R,2S)-2-[4-(1-(R)-hydroxyhexyl)phenyl]-5-oxo-cyclopentaneheptanoic acid [RSR]

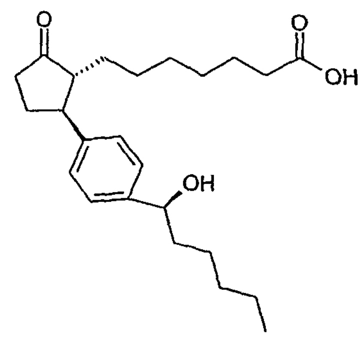

(1R,2S)-2-[4-(1-(S)-hydroxyhexyl)phenyl]-5-oxo-cyclopentaneheptanoic acid [RSS]

The present applicants have been able to separate the four major stereoisomers from each other and have determined their relative activities. However, initial attempts to separate these stereoisomers were not successful.

Attempts were carried out on a mixture of all the stereoisomers in their acid form using chiral HPLC using a variety of commercially available stationary phases, but these were unsuccessful.

Many attempts at separation were carried out on the two mixtures of esters produced in example 1 below, using chiral HPLC on a variety of commercially available stationary phases and mobile phases, but at best this method was successful on an analytical level and separation was not possible on a preparative scale.

Finally, however, attempts to separate the stereoisomers as esters was successful as is described below in Example 2.

The present inventors have also devised a stereoselective synthesis route for the stereoisomers of interest.

*Summary of the Invention*

In a first aspect, the invention provides a compound selected from one of the following forms:

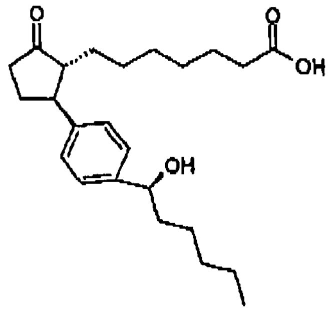

(1R,2S)-2-[4-(1-(S)-hydroxyhexyl)phenyl]-5-oxo-cyclopentaneheptanoic acid
[RSS]

; or

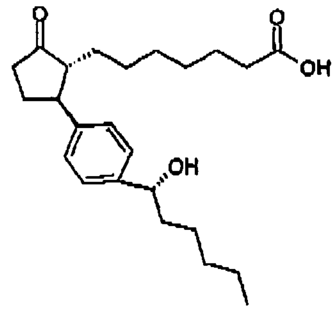

(1R,2S)-2-[4-(1-(R)-hydroxyhexyl)phenyl]-5-oxo-cyclopentaneheptanoic acid
[RSR]

or a salt or solvate thereof.

In a second aspect, the invention provides *trans*-2-[4-(1-hydroxyhexyl)phenyl]-5-oxo-cyclopentaneheptanoic acid, of which at least 90% by weight is selected from one of the following forms :

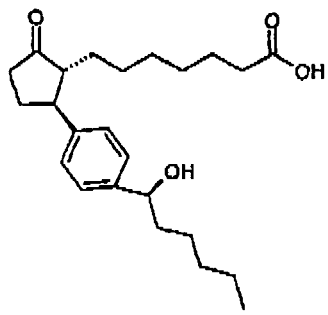

(1R,2S)-2-[4-(1-(S)-hydroxyhexyl)phenyl]-5-oxo-cyclopentaneheptanoic acid
[RSS]

; or

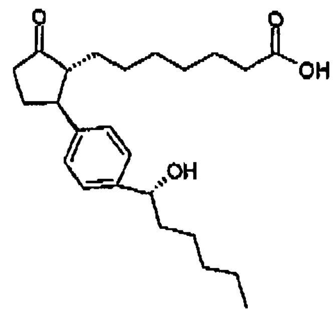

(1R,2S)-2-[4-(1-(R)-hydroxyhexyl)phenyl]-5-oxo-cyclopentaneheptanoic acid
[RSR]

or a salt or solvate thereof

In a third aspect, the present invention provides 2-[4-(1-hydroxyhexyl)phenyl]-5-oxo-yclopentaneheptanoic acid, of which at least 80% by weight is in one of the following forms:

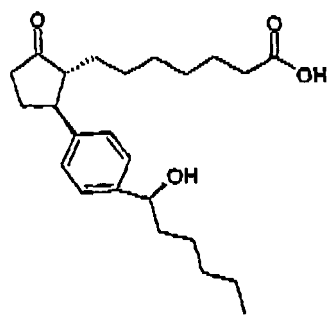

(1R,2S)-2-[4-(1-(S)-hydroxyhexyl)phenyl]-5-oxo-cyclopentaneheptanoic acid
[RSS]

; or

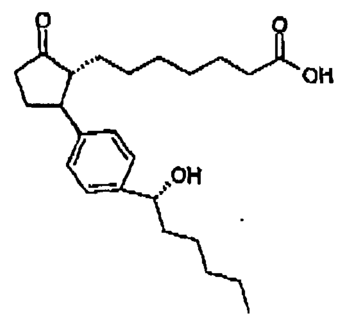

(1R,2S)-2-[4-(1-(R)-hydroxyhexyl)phenyl]-5-oxo-cyclopentaneheptanoic acid
[RSR]

or a salt or solvate thereof.

A further aspect of the present invention provides a compound of any one of the first to third aspects, or a

pharmaceutically acceptable salt thereof for use in a method of therapy.

Another aspect of the present invention provides a pharmaceutical composition comprising a compound of any one of the first to third aspects, or a pharmaceutically acceptable salt thereof together with a pharmaceutically acceptable carrier or diluent.

A further aspect of the present invention provides the use of a compound of any one of the first to third aspects, or a pharmaceutically acceptable salt thereof in the preparation of a medicament for the treatment of a condition alleviated by agonism of an $EP_2$ receptor. This aspect also provides a compound of any one of the first to third aspects, or a pharmaceutical acceptable salt thereof, for use in a method of treatment of a condition alleviated by agonism of $EP_2$ receptor.

Other aspects of the present invention provide methods of synthesizing the compounds of the invention, or relevant intermediates, by the methods set out below.

Conditions which can be treated by agonism of an $EP_2$ receptor are discussed above, and particularly include dysmenorrhoea, pre-term labour, glaucoma, osteoporosis, asthma, allergy, bone disease, fracture repair, infertility, male sexual dysfunction, female sexual dysfunction, periodontal disease, gastric ulcer and renal disease.

EP receptor agonists are known to be able to inhibit T-cell activation and the release of pro-inflammatory cytokines, although the EP receptor involved in mediating these effects in human T-cells has not been previously defined. The present inventors have discovered that $EP_2$ agonists inhibit human T-cell activation (proliferation) and inhibit the release of multiple pro-inflammatory cytokines including interleukin 2 (IL-2) tumour necrosis factor ($TNF_\alpha$) and interferon gamma (IFN$\gamma$). This profile of activity strongly suggests that $EP_2$ receptor agonists will be useful in treating immune and inflammatory disorders, including but not limited to psoriasis, psoriatic arthritis, dermatitis, rheumatoid arthritis, transplant rejection, inflammatory bowel disease, systemic lupus erythematosus, graves disease, scleroderma, multiple sclerosis, Type I diabetes, and transplant rejection, and in particular psoriasis (Griffiths, C., Current Drugs Targets - Inflammation & Allergy, 3, 157-161, (2004); Lebwohl, M., Lancet, 361, 1197-1204 (2003); Salim, A- & Emerson, R., Curr. Open. Investig. Drug, 2(11), 1546-8 (2001)). Therefore, a further condition which can be alleviated by agonism of an $EP_2$ receptor is psoriasis.

Furthermore, the present inventors have also shown that $EP_2$ receptor agonists inhibit the release of the pro-inflammatory cytokine, TNF$\alpha$ from human monocytes and alveolar macrophages. This profile of activity adds further evidence to the view that that $EP_2$ receptor agonists will be useful in treating immune and inflammatory disorders and in particular, inflammatory lung diseases (including, but not limited to: asthma, chronic obstructive pulmonary disease, acute respiratory distress syndrome, pulmonary fibrosis and cystic fibrosis))

Furthermore, aspects of the present invention relate to the use of $EP_2$ agonists as provided on the first to third aspects to treat conditions ameliorated by the inhibition of IL-2 and/or IFN$\gamma$ production and the use of an $EP_2$ agonist in the preparation of a medicament for the treatment of a condition alleviated by inhibition of IL-2 production.

In some embodiments, the compounds described above may show selectivity for $EP_2$ receptors relative to the other three EP receptors, i.e. $EP_1$, $EP_3$ and $EP_4$. This selectivity allows for targeting of the effect of the compounds of the invention, with possible benefits in the treatment of certain conditions.

The invention will be described with reference to the attached figures, in which:

Figure 1a shows the CD spectrum of prostaglandin $E_2$ ($PGE_2$) (0.7 mg/mL) in ethanol using a 1.0 cm pathlength cuvette.

Figure 1b shows the UV spectrum of $PGE_2$ (0.7 mg/mL) in ethanol using a 1.0 cm pathlength cuvette.

Figure 2a shows the CD spectrum of (R)-1-(4-bromophenyl)hexan-1-ol (R-BPH) (solid line in figure) (0.7 mg/mL) and (S)-1-(4-bromophenyl)hexan-1-ol (S-BPH) (dashed line in figure) (0.7 mg/mL) in ethanol using a 1.0 cm pathlength cuvette.

Figure 2b shows the UV spectrum of R-BPH (solid line in figure) (0.7 mg/mL) and S-BPH (dashed line in figure) (0.7 mg/mL) in ethanol using a 1.0 cm pathlength cuvette (N.b. solid and dashed lines almost overlie each other in this figure).

Figure 3a shows the CD spectrum of each of the four *trans*-stereoisomers of Example 4 (compounds A, C, E and G) (all 19 mg/mL) in ethanol using a 0.1 cm pathlength cuvette.

Figure 4 shows the variation in percentage of $[^3H]PGE_2$ displaced with concentration of five test compounds in an assay of binding ability to human $EP_2$ receptors;

Figure 5 shows the variation in concentration of cAMP following stimulation by five test compounds in an assay of

human $EP_2$ receptor stimulation;

Figure 6 shows the effect on human myometrial activity of AH13205;

Figure 7 shows the variation in % inhibition of electrical field stimulation (EFS) induced contractions with concentrations of AH13205 and delivery vehicle or delivery vehicle alone in an assay of human myometrial activity;

Figure 8 shows the variation in % of control electrical field stimulation (EFS) induced contractions with concentrations of three test compounds in an assay of human myometrial activity;

Figure 9 shows the variation in IL-2 production with concentration of 4 test compounds in a lymphocyte assay;

Figure 10 shows the variation of IL-2 production with concentration of 3 $EP_2$ receptor agonists in a lymphocyte assay;

Figure 11 shows the variation of Interferon gamma release with concentration of 3 $EP_2$ receptor agonists in a lymphocyte assay;

Figure 12 shows the variation of TNFα production in response to 3 $EP_2$ receptor agonists in a lymphocyte assay;

Figure 13 shows the variation of cell proliferation in response to 3 $EP_2$ receptor agonists in a lymphocyte assay;

Figure 14 shows the variation of TNFα production in response to 3 test compounds in a monocyte assay;

Figure 15 shows the variation of TNFα production in response to 2 test compounds in an alveolar macrophage assay.

*Definitions*

**Includes Other Forms**

Unless otherwise specified, included in the above are the well known ionic, salt, solvate, and protected forms of these substituents. For example, a reference to carboxylic acid (-COOH) also includes the anionic (carboxylate) form ($-COO^-$), a salt or solvate thereof, as well as conventional protected forms. Similarly, a reference to a hydroxyl group also includes the anionic form ($-O^-$), a salt or solvate thereof, as well as conventional protected forms of a hydroxyl group.

**Salts, Solvates and Protected Forms**

It may be convenient or desirable to prepare, purify, and/or handle a corresponding salt of the active compound, for example, a pharmaceutically-acceptable salt. Examples of pharmaceutically acceptable salts are discussed in Berge, et al., J. Pharm. Sci., 66, 1-19 (1977).

For example, if the compound is anionic, or has a functional group which may be anionic (e.g. -COOH may be $-COO^-$), then a salt may be formed with a suitable cation. Examples of suitable inorganic cations include, but are not limited to, alkali metal ions such as $Na^+$ and $K^+$, alkaline earth cations such as $Ca^{2+}$ and $Mg^{2+}$ , and other cations such as $Al^{3+}$. Examples of suitable organic cations include, but are not limited to, ammonium ion (i.e. $NH_4^+$) and substituted ammonium ions (e.g. $NH_3R^+$, $NH_2R_2^+$, $NHR_3^+$, $NR_4^+$). Examples of some suitable substituted ammonium ions are those derived from: ethylamine, diethylamine, dicyclohexylamine, triethylamine, butylamine, ethylenediamine, ethanolamine, diethanolamine, piperazine, benzylamine, phenylbenzylamine, choline, meglumine, and tromethamine, as well as amino acids, such as lysine and arginine. An example of a common quaternary ammonium ion is $N(CH_3)_4^+$.

It may be convenient or desirable to prepare, purify, and/or handle a corresponding solvate of the active compound. The term "solvate" is used herein in the conventional sense to refer to a complex of solute (e.g., active compound, salt of active compound) and solvent. If the solvent is water, the solvate may be conveniently referred to as a hydrate, for example, a mono-hydrate, a di-hydrate, a tri-hydrate, etc.

It may be convenient or desirable to prepare, purify, and/or handle the active compound in a chemically protected form. The term "chemically protected form" is used herein in the conventional chemical sense and pertains to a compound in which one or more reactive functional groups are protected from undesirable chemical reactions under specified conditions (e.g. pH, temperature, radiation, solvent, and the like). In practice, well known chemical methods are employed to reversibly render unreactive a functional group, which otherwise would be reactive, under specified conditions. In a chemically protected form, one or more reactive functional groups are in the form of a protected or protecting group (also known as a masked or masking group or a blocked or blocking group). By protecting a reactive functional group,

reactions involving other unprotected reactive functional groups can be performed, without affecting the protected group; the protecting group may be removed, usually in a subsequent step, without substantially affecting the remainder of the molecule. See, for example, Protective Groups in Organic Synthesis (T. Green and P. Wuts; 3rd Edition; John Wiley and Sons, 1999).

A wide variety of such "protecting", "blocking", or "masking" methods are widely used and well known in organic synthesis. For example, a compound which has two nonequivalent reactive functional groups, both of which would be reactive under specified conditions, may be derivatized to render one of the functional groups "protected," and therefore unreactive, under the specified conditions; so protected, the compound may be used as a reactant which has effectively only one reactive functional group. After the desired reaction (involving the other functional group) is complete, the protected group may be "deprotected" to return it to its original functionality.

For example, a hydroxy group may be protected as an ether (-OR) or an ester (-OC(=O)R), for example, as: a t-butyl ether; a benzyl, benzhydryl (diphenylmethyl), or trityl (triphenylmethyl) ether; a trimethylsilyl or t-butyldimethylsilyl ether; or an acetyl ester (-OC(=O)$CH_3$, -OAc).

For example, a carboxylic acid group may be protected as an ester for example, as: an $C_{1-7}$ alkyl ester (e.g., a methyl ester; a t-butyl ester); a $C_{1-7}$ haloalkyl ester (e.g., a $C_{1-7}$ trihaloalkyl ester); a tri$C_{1-7}$ alkylsilyl-$C_{1-7}$ alkyl ester; or a $C_{5-20}$ aryl-$C_{1-7}$ alkyl ester (e.g. a benzyl ester; a nitrobenzyl ester); or as an amide, for example, as a methyl amide.

### Prodrugs

It may be convenient or desirable to prepare, purify, and/or handle the active compound in the form of a prodrug. The term "prodrug," as used herein, pertains to a compound which, when metabolised (e.g., in vivo), yields the desired active compound. Typically, the prodrug is inactive, or less active than the active compound, but may provide advantageous handling, administration, or metabolic properties.

Unless otherwise specified, a reference to a particular compound also include prodrugs thereof.

For example, some prodrugs are esters of the active compound (e.g., a physiologically acceptable metabolically labile ester). During metabolism, the ester group (-C(=O)OR) is cleaved to yield the active drug. Such esters may be formed by esterification, for example, of any of the carboxylic acid groups (-C(=O)OH) in the parent compound, with, where appropriate, prior protection of any other reactive groups present in the parent compound, followed by deprotection if required.

Examples of such metabolically labile esters include those of the formula -C(=O)OR wherein R is:
$C_{1-7}$ alkyl
(e.g., -Me, -Et, -nPr, -iPr, -nBu, -sBu, -iBu, -tBu);
$C_{1-7}$ aminoalkyl
(e.g., aminoethyl; 2-(N,N-diethylamino)ethyl;
2-(4-morpholino)ethyl);
$C_{1-7}$ hydroxy or polyhydroxt alkyl
(e.g. 2-hydroxyethyl, 2.3-dihydroxypropyl (glyceryl))and acyloxy-$C_{1-7}$alkyl (e.g., acyloxymethyl; acyloxyethyl; pivaloyloxymethyl; acetoxymethyl; 1-acetoxyethyl; 1-(1-methoxy-1-methyl)ethyl-carbonxyloxyethyl; 1-(benzoyloxy)ethyl; isopropoxy-carbonyloxymethyl; 1-isopropoxy-carbonyloxyethyl; cyclohexyl-carbonyloxymethyl; 1-cyclohexyl-carbonyloxyethyl; cyclohexyloxy-carbonyloxymethyl; 1-cyclohexyloxy-carbonyloxyethyl; (4-tetrahydropyranyloxy) carbonyloxymethyl; 1-(4-tetrahydropyranyloxy)carbonyloxyethyl; (4-tetrahydropyranyl)carbonyloxymethyl; and 1-(4-tetrahydropyranyl)carbonyloxyethyl).

Also, some prodrugs are activated enzymatically to yield the active compound, or a compound which, upon further chemical reaction, yields the active compound (for example, as in ADEPT, GDEPT, LIDEPT, etc.). For example, the prodrug may be a sugar derivative or other glycoside conjugate, or may be an amino acid ester derivative.

### Treatment and Therapy

The term "treatment", as used herein in the context of treating a condition, pertains generally to treatment and therapy, whether of a human or an animal (e.g. in veterinary applications), in which some desired therapeutic effect is achieved, for example, the inhibition of the progress of the condition, and includes a reduction in the rate of progress, a halt in the rate of progress, amelioration of the condition, and cure of the condition. Treatment as a prophylactic measure (i.e. prophylaxis) is also included.

The term "therapeutically-effective amount", as used herein, pertains to that amount of an active compound, or a material, composition or dosage form comprising an active compound, which is effective for producing some desired therapeutic effect, commensurate with a reasonable benefit/risk ratio, when administered in accordance with a desired treatment regimen. Suitable dose ranges will typically be in the range of from 0.01 to 20 mg/kg/day, preferably from 0.1 to 10 mg/kg/day, although the dose may be as low as from about 0.00001 to 1 mg/day in the case of the topical ocular

administration.

### Compositions and their administration

Compositions may be formulated for any suitable route and means of administration. Pharmaceutically acceptable carriers or diluents include those used in formulations suitable for oral, rectal, nasal, topical (including buccal, ocular and sublingual), vaginal or parenteral (including subcutaneous, intramuscular, intravenous, intradermal, intrathecal and epidural) administration. The formulations may conveniently be presented in unit dosage form and may be prepared by any of the methods well known in the art of pharmacy. Such methods include the step of bringing into association the active ingredient with the carrier which constitutes one or more accessory ingredients. In general the formulations are prepared by uniformly and intimately bringing into association the active ingredient with liquid carriers or finely divided solid carriers or both, and then, if necessary, shaping the product.

For solid compositions, conventional non-toxic solid carriers include, for example, pharmaceutical grades of mannitol, lactose, cellulose, cellulose derivatives, starch, magnesium stearate, sodium saccharin, talcum, glucose, sucrose, magnesium carbonate, and the like may be used. The active compound as defined above may be formulated as suppositories using, for example, polyalkylene glycols, acetylated triglycerides and the like, as the carrier. Liquid pharmaceutically administrable compositions can, for example, be prepared by dissolving, dispersing, etc, an active compound as defined above and optional pharmaceutical adjuvants in a carrier, such as, for example, water, saline aqueous dextrose, glycerol, ethanol, polyoxol esters and the like, to thereby form a solution or suspension. If desired, the pharmaceutical composition to be administered may also contain minor amounts of non-toxic auxiliary substances such as wetting or emulsifying agents, pH buffering agents and the like, for example, sodium acetate, sorbitan monolaurate, triethanolamine sodium acetate, sorbitan monolaurate, triethanolamine oleate, etc. Actual methods of preparing such dosage forms are known, or will be apparent, to those skilled in this art; for example, see Remington's Pharmaceutical Sciences, 20th edition, pub. Lippincott, Williams & Wilkins, 2000. The composition or formulation to be administered will, in any event, contain a quantity of the active compound(s) in an amount effective to alleviate the symptoms of the subject being treated.

Dosage forms or compositions containing active ingredient in the range of 0.25 to 95% with the balance made up from non-toxic carrier may be prepared.

For oral administration, a pharmaceutically acceptable non-toxic composition is formed by the incorporation of any of the normally employed excipients, such as, for example, pharmaceutical grades of mannitol, lactose, cellulose, cellulose derivatives, sodium crosscarmellose, starch, magnesium stearate, sodium saccharin, talcum, glucose, sucrose, magnesium carbonate, and the like. Such compositions take the form of solutions, suspensions, tablets, pills, capsules, powders, sustained release formulations and the like. Such compositions may contain 1%-95% active ingredient, more preferably 2-50%, most preferably 5-8%.

Parenteral administration is generally characterized by injection, either subcutaneously, intramuscularly or intravenously. Injectables can be prepared in conventional forms, either as liquid solutions or suspensions, solid forms suitable for solution or suspension in liquid prior to injection, or as emulsions. Suitable excipients are, for example, water, saline, dextrose, glycerol, ethanol or the like. In addition, if desired, the pharmaceutical compositions to be administered may also contain minor amounts of non-toxic auxiliary substances such as wetting or emulsifying agents, pH buffering agents and the like, such as for example, sodium acetate, sorbitan monolaurate, triethanolamine oleate, triethanolamine sodium acetate, etc.

The percentage of active compound contained in such parental compositions is highly dependent on the specific nature thereof, as well as the activity of the compound and the needs of the subject. However, percentages of active ingredient of 0.1% to 10% in solution are employable, and will be higher if the composition is a solid which will be subsequently diluted to the above percentages. Preferably, the composition will comprise 0.2-2% of the active agent in solution.

Formulations suitable for transdermal administration include gels, pastes, ointments, creams, lotions, and oils, as well as patches, adhesive plasters, bandages, dressings, depots, and reservoirs.

Ointments are typically prepared from the active compound and a paraffinic or a water-miscible ointment base.

Creams are typically prepared from the active compound and an oil-in-water cream base. If desired, the aqueous phase of the cream base may include, for example, at least about 30% w/w of a polyhydric alcohol, i.e., an alcohol having two or more hydroxyl groups such as propylene glycol, butane-1,3-diol, mannitol, sorbitol, glycerol and polyethylene glycol and mixtures thereof. The topical formulations may desirably include a compound which enhances absorption or penetration of the active compound through the skin or other affected areas. Examples of such dermal penetration enhancers include dimethylsulfoxide and related analogues. Formulations suitable for vaginal administration may be presented as pessaries, tampons, creams, gels, pastes, foams or spray formulations containing in addition to the active compound, such carriers as are known in the art to be appropriate.

Eye drops, for topical ocular administration preferably comprise between 0.001 and 20% of the active agent

with the remainder made up from well known carriers such as water or saline, or other additives as discussed below.

Typical ocular compositions may include:

(a) antimicrobial preservatives - suitable preservatives include: benzalkonium chloride, thimerosal, chlorobutanol, methyl paraben, propyl paraben, phenylethyl alcohol, edetate disodium, sorbic acid, onamer, or other agents known to those skilled in the art. Such preservatives are typically employed at a concentration between about 0.001% and about 1.0% by weight;
(b) solubilising agents, such as Polysorbate 20, 60 and 80; Pluronic F-68, F-84 and P-103; Tyloxapol; Cremophor; sodium dodecyl sulfate; glycerol; PEG 400; propylene glycol; cyclodextrins; or other agents known to those skilled in the art. Such co-solvents are typically employed at a concentration between about 0.01% and about 2% by weight; and
(c) viscosity agents, such as polyvinyl alcohol, polyvinyl pyrrolidone, methyl cellulose, hydroxy propyl methylcellulose, hydroxyethyl cellulose, carboxymethyl cellulose, hydroxy propyl cellulose or other agents known to those skilled in the art. Such agents are typically employed at a concentration between about 0.01% and about 2% by weight.

Other optional components, sometimes termed 'inactive ingredients', include sodium chloride, sodium dihydrogen phosphate monohydrate and/or anhydrous, polyoxyl 40 hydrogenated caster oil, tromethamine, boric acid, mannitol, edetate disodium, sodium hydroxide and/or hydrochloric acid to adjust pH and purified water.

Ocular formulations containing prostaglandins are described in, amongst others: US 5,889,052; US 4,599,353; US 6,011,062; US 6,235,781; US 5,849,792; US 5,631,287, which are herein incorporated by reference.

**General Synthesis Methods**

Compounds of formula 1:

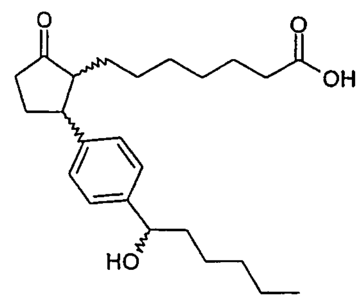

Formula 1

wherein 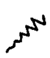 represents a defined stereochemistry at each chiral centre, and the groups on the pentanone are *trans* to one another, may be synthesised from compounds of formula 2:

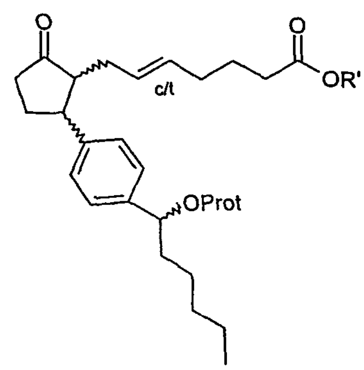

Formula 2

having the same stereochemistry at each chiral centre, wherein R' represents a $C_{1-7}$ alkyl group (a monovalent moiety obtained by removing a hydrogen atom from a carbon atom of a hydrocarbon compound having from 1 to 7 carbon atoms, e.g. methyl ($C_1$), ethyl ($C_2$), propyl ($C_3$), butyl ($C_4$), pentyl ($C_5$), hexyl ($C_6$), heptyl ($C_7$)) by reduction of the double bond and deprotection of the acid and alcohol using standard techniques e.g. the reduction may be carried out with hydrogen, palladium on charcoal in a solvent such as ethyl acetate at normal temperature and pressure. A particularly preferred alcohol protecting group is a silyl group, such as tert-butyldimethylsilyl (TBDMS), which can be removed, for example, with aqueous acid and a co-solvent, which conditions may also deprotect the acid group. These reactions may be carried out in either order. The double bond may be either in the *cis*- or *trans*- orientation, or a mixture of these.

Compounds of formula 2 may be synthesised by trapping an enolate of formula 3:

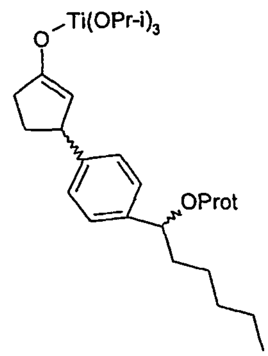

Formula 3

having the same stereochemistry at each of the two chiral centres, with a compound of formula 4:

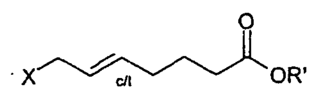

Formula 4

wherein X is a leaving group, such as halide or mesylate, and R' is as in formula 2, in the presence of a strong base, such as Li Oi-Pr at room temperature.

The four enolates of formula 3 may be generated from cyclopent-2-enone (formula 5):

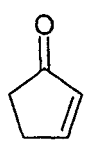

Formula 5

by reacting it with a compound of formula 6:

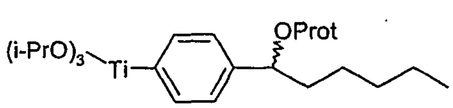

Formula 6

having the same stereochemistry at the chiral centre, in the presence of a transition metal catalyst, preferably Rh(I), in the presence of a chiral ligand, such as BINAP, (2,2'-Bis(diphenylphosphino)-1,1'-binaphthyl). Using *S*-BINAP would yield enolates of formula 3a:

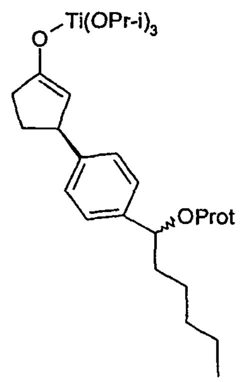

Formula 3a

,

whereas using *R*-BINAP would yield enolates of formula 3b:

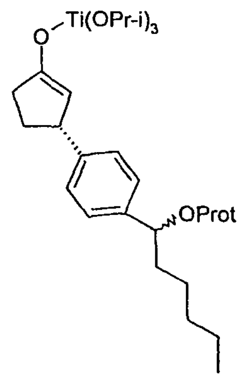

Formula 3b

The compounds of formula 6 may be generated in situ from the reaction of compounds of formula 7:

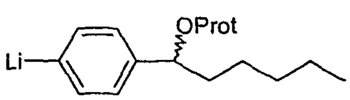

Formula 7

having the same stereochemistry at the chiral centre, with $ClTi(Oi\text{-}Pr)_3$, before the reaction of compounds of formulae 5 and 6. This reaction may be carried generally in accordance with the methods described in Hayashi, T., et al., JACS, 124, 12102-12103 (2002), such as 1.6 equivalents of the compound of formula 6 to the compound of formula 5, with 3% of the catalyst in tetrahydrofuran at 20°C for 1 hour under an inert atmosphere.

Compounds of formula 7 can be generated from the corresponding bromo compound of formula 8:

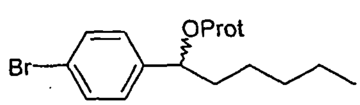

Formula 8

with the same stereochemistry at the chiral centre, by treating with an alkyl lithium, in a solvent, for example THF.

Compounds of formula 8 are made by protecting compounds of formula 9:

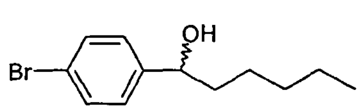

Formula 9

with the same stereochemistry at the chiral centre, using standard conditions, which retain the stereochemistry of the chiral centre, e.g. reaction with TBDMSCl.

The single stereoisomers of compound 9 can by made from a compound of formula 10:

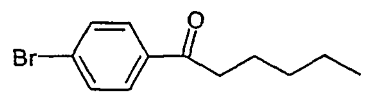

Formula 10

by either enantioselective reduction (e.g. see Brown, H.C., et al. J. Am. Chem. Soc., 110, 1539-1546 (1988)), or by reduction to the racemate of compound 9 followed by optical resolution.

Compounds of formula 4 are known from the synthesis of natural prostaglandins, e.g. Suzuki, M., et al., J. Am.Chem.Soc., 107, 3348-3349 (1985), and may be synthesised by a variety of routes.

One route, based on a route disclosed in Taber, D.F., et al., J. Org. Chem., 62, 194-198 (1997), is as follows:

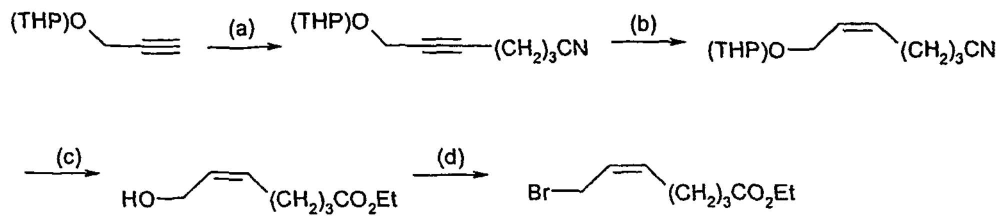


(a) BuLi, $Br(CH_2)_3Br$ (80%); NaCN, DMSO (99%);
(b) $Ni(OAc)_2$, $NaBH_4$ (87%);
(c) Dowex, MeOH (78%); NaOH, EtOH, $BF_3$ (84%);
(d) $CBr_4$, $Ph_3P$ (76%)
(34% th.; 57% wt. overall)

Other possible methods use different alkylating agents for the propargyl alcohol (and are illustrated below), but preparation of the alkylating agents require additional step. An example of this is alkylation of the ortho ester of bromobutyrate (Patterson, J.W., et al., Synthesis, 1985, 337-338). The ortho ester of 5-hexynoic acid has been reacted with BuLi/formaldehyde to give the same intermediate (Syn.Comms. 1989, p.1509). A further possible route may involve the direct reaction of 5-hexynoic acid (commercially available, Aldrich) with base and formaldehyde.

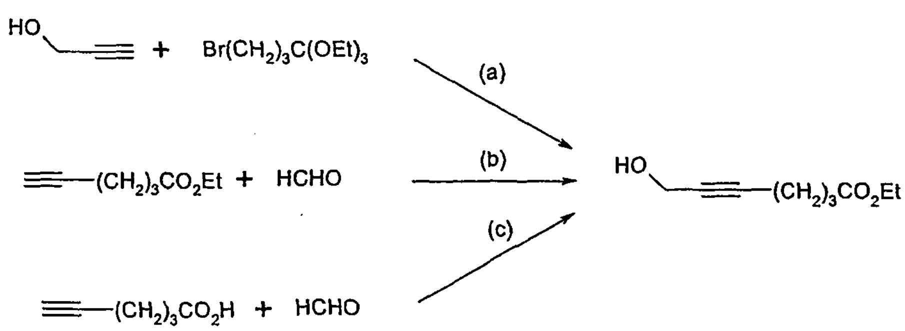


(a) BuLi
(b) BuLi
(c) base

An alternative route to the four compounds of formula 1 is from compounds of formula 11:

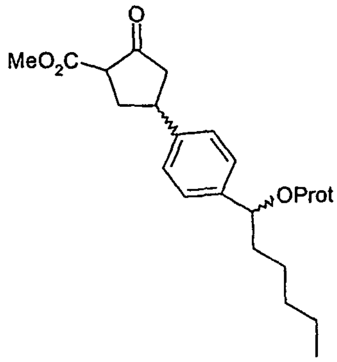

Formula 11

where the chiral centres have the same stereochemistry, by reaction first with sodium hydride and then with strong base, such as potassium amide or butyl lithium, to form the dianion, (Weiler, L., J. Am.Chem. Soc., 92, 6702-6704 (1970) and see, for example, Modern Synthetic Reactions, 2nd Edition 1972, H.O. House, p. 553), which can then be reacted with haloheptanoate to give the substituted ketoester (Huckin, S.N. and Weiler, L., Can. J. Chem., 52, 2157 (1974)), which subsequently can be hydrolysed and decarboxylated using standard conditions, e.g. treating with lithium iodide in collidine or treating with sodium cyanide in DMSO (see Modern Synthetic Reactions, 2nd Edition 1972, H.O. House, p. 511-517). It may be necessary to replace the haloheptanoate with a more reactive alkylating agent such as the allylic halide of Formula 4, followed by reduction of the double bond.

The compounds of formula 11 can be synthesised from compounds of formula 12:

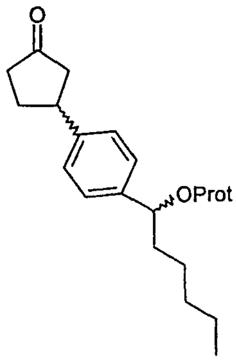

Formula 12

having the same stereochemistry at each of the two chiral centres, by reaction with dimethyl carbonate and a base, such as sodium hydride, in a solvent such as toluene or THF, with mild heating.

The compounds of formula 12 can be synthesised from cyclopent-2-enone (formula 5):

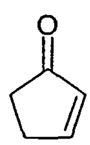

Formula 5

by boronic acid addition (see Takaya, Y., et al., J. Am. Chem. Soc., 120, 5579-5580 (1998) and Hayashi, T., Synlett, SI, 879-887 (2001)) of a compound of formula 13:

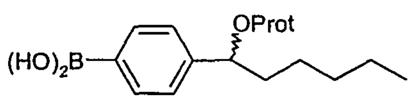

Formula 13

with the same stereochemistry at the chiral centre, in the presence of a transition metal catalyst, preferably Rh(I), in the presence of a chiral ligand, preferably BINAP. Suitable conditions include the use of 3% catalyst and chiral ligand in aqueous dioxane at 60°C for 20 hours.

By analogy with established chemical precedent, use of S-BINAP yields compounds of formula 12a:

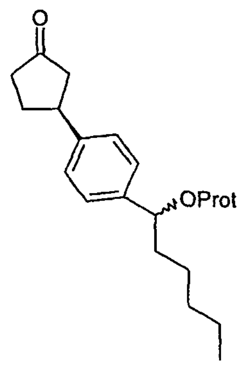

Formula 12a

whilst using R-BINAP yields compounds of formula 12b:

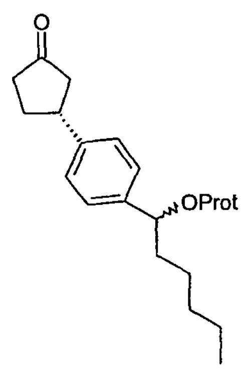

Formula 12b

Compounds of formula 13 may be generated from compounds of formula 8, with the same stereochemistry at the chiral centre, by standard techniques. Such techniques include first treatment with a lithium exchange reagent, for example t-butyl lithium, in a solvent, for example THF, at a suitable temperature (for butyl lithium in THF, -78°C). This is followed by treatment with an appropriate boron reagent, for example $B(O^{i}\text{-}Pr)_3$ followed by hydrolysis, e.g. by potassium hydroxide (Thompson, W.J. and Gaudino, J., J. Org. Chem., 49, 5237-5243 (1984)).

An alternative route from compounds of formula 13 to compounds of formula 11 where the chiral centres have the same stereochemistry is reaction of compounds of formula 13 with the methylcarboxy substituted cyclopent-2-enone of formula 14:

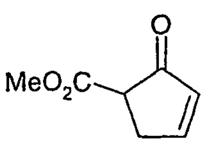

Formula 14

by boronic acid addition, in the presence of a transition metal catalyst, preferably Rh(I), in the presence of a chiral ligand, preferably BINAP. Suitable conditions include the use of 3% catalyst and chiral ligand in aqueous dioxane at 60°C for 20 hours, i.e. similar reaction conditions used for the coupling of compound 5 with compounds of formula 13.

A further alternative route to the four compounds of formula 1 is from compounds of formula 15:

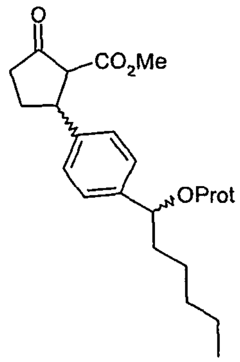

Formula 15

where the chiral centres have the same stereochemistry, by reaction with strong base, such as sodium hydride, e.g. in DMF, to form a monoanion, which can then be reacted with haloheptanoate to give the substituted ketoester, which subsequently can be hydrolysed and decarboxylated using standard conditions, e.g. treating with lithium iodide in collidine or treating with sodium cyanide in DMSO (see Modern Synthetic Reactions, 2nd Edition 1972, H.O. House, p. 511-517). The trans arrangement on the cyclopentanone arises due to steric hindrance.

The compounds of formula 15 can be synthesized by coupling compounds of formula 13, with the same stereochemistry at the chiral centre, with the methylcarboxy substituted cyclopent-2-enone of formula 16:

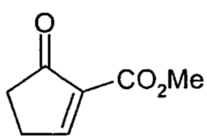

Formula 16

(Funk, R.L., et al., J. Am. Chem. Soc, 115, 8849-8850 (1993)), in the presence of a transition metal catalyst, preferably Rh(I), in the presence of a chiral ligand, preferably BINAP. Suitable conditions include the use of 3% catalyst and chiral ligand in aqueous dioxane at 60°C for 20 hours, i.e. similar reaction conditions used for the coupling of compound 5 with compounds of formula 13.

Alternatively, the stereoselective synthesis of (1R,2S)-2-[4-(1-(S)-hydroxyhexyl)phenyl]-5-oxo-cyclopentaneheptanoic acid (formula 17) may be achieved by the following general synthesis route.

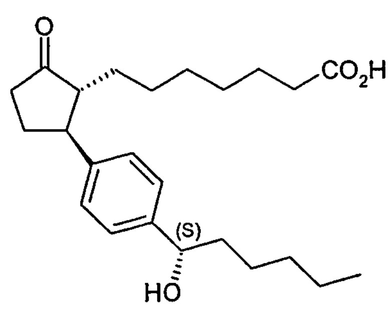

Formula 17

A compound of formula 17 may be formed from compound 18 in which the -OH group on the 4-hexyl-phenyl side chain is protected by an alcohol-protecting group.

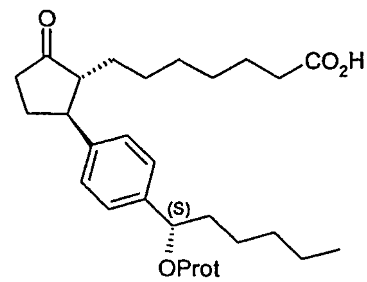

Formula 18

As mentioned previously, a particularly preferred alcohol-protecting group is a silyl group, such as tert-butyldimethylsilyl (TBDMS), which can be removed, for example, with aqueous acid and a co-solvent, for example THF.

Compounds of formula 18 may be formed from compounds of formula 19 by removal of the carboxymethyl group and hydrolysis of the heptanoate ester. This may be achieved, for example, by reaction with lithium iodide and 2,4,6-trimethyl pyridine (collidine).

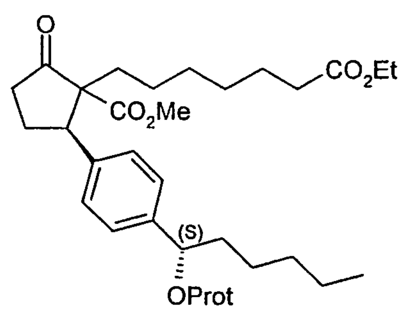

Formula 19

Compound 19 can be synthesised by addition of the heptyl-ethyl ester side chain to a compound of formula 20.

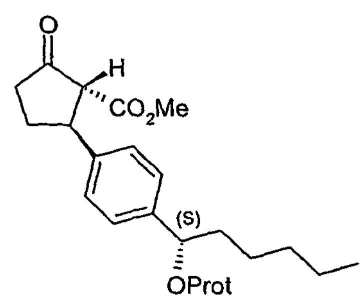

Formula 20

This can be achieved by the treatment of a compound of formula 20 with a suitable base in order to remove the hydrogen atom adjacent to the -$CO_2$Me group. The preferred base for this reaction is NaH in anhydrous solvent, such as DME.

Subsequent addition of ethyl heptanoate, activated at the 7-position, to the resultant compound gives a compound of formula 19. Preferably, the ethyl heptanoate is activated with a halogen atom in the 7- position. More preferably, this halogen atom is a bromine atom. It may also be necessary to incude a catalyst during the addition of the activated ethyl heptanoate. Such a catalyst is preferably sodium iodide.

Compounds of formula 20 maybe formed from compounds of formula 21 via a transesterification reaction.

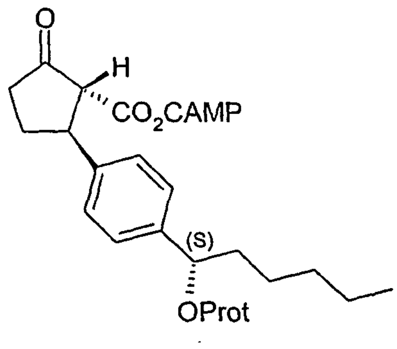

CAMP =

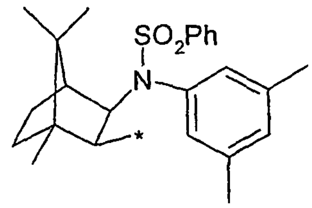

Formula 21

Formation of the methyl ester of compound 20 may be achieved by heating compound 21 with methanol in a sealed vessel.

Compounds of formula 21 can be formed by 1,4-addition to an unsaturated carbonyl compound of formula 22.

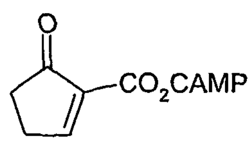

Formula 22

This can be achieved by reaction of an organometallic reagent of formula 23 with a copper (I) compound followed by addition to a compound of formula 22.

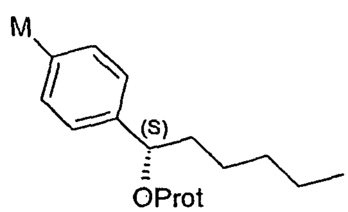

Formula 23

Where M in formula 23 represents an element which is less electronegative than copper. Preferably M is Li, MgX, $BR_2$ and ZnX, where X is a halogen atom.

The copper (I) reagent used in the coupling reaction of compounds of formulae 22 and 23 is preferably an anionic cuprate and is more preferably LiCu-(2-Th)CN (known as lithium 2-thienylcyanocuprate).

A compound of formula 23 may be formed by reaction of a halide of formula 24 by standard organometallic formation reactions.

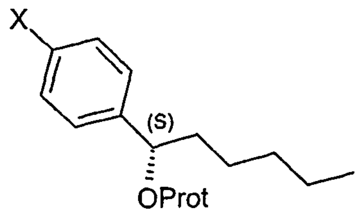

Formula 24

In formula 24, X is a halogen atom and is preferably I or Br.

Compounds of formula 22 may be formed from readily available starting materials by the method described in Tetrahedron 1996, 52, 971-986.

**Acronyms**

For convenience, many chemical moieties are represented using well known abbreviations, including but not limited to, methyl (Me), ethyl (Et), n-propyl (nPr), iso-propyl (iPr), n-butyl (nBu), sec-butyl (sBu), iso-butyl (iBu), tert-butyl (tBu), n-hexyl (nHex), cyclohexyl (cHex), phenyl (Ph), biphenyl (biPh), benzyl (Bn), naphthyl (naph), methoxy (MeO), ethoxy (EtO), benzoyl (Bz), tetrahydropyranyl (THP) and acetyl (Ac).

For convenience, many chemical compounds are represented using well known abbreviations, including but not limited to, methanol (MeOH), ethanol (EtOH), iso-propanol (i-PrOH), methyl ethyl ketone (MEK), ether or diethyl ether ($Et_2O$), acetic acid (AcOH), dichloromethane (methylene chloride, DCM), acetonitrile (ACN), trifluoroacetic acid (TFA), dimethylformamide (DMF), tetrahydrofuran (THF), ethyl acetate (EA), 1.2-dimehoxyethane (DME) and dimethylsulfoxide (DMSO).

*Selectivity*

The selectivity of the compound for agonising $EP_2$ receptors over the other EP receptors (i.e. $EP_1$, $EP_3$, $EP_4$) can be quantified by dividing the Ki for $EP_2$ (see below) by the Ki for the other EP receptors (see below). The resulting inverse ratio is preferably 10 or more, more preferably 100 or more.

**Examples**

Nmr spectra were recorded on either a Bruker AV300 or Bruker DPX400. Mass spectra were recorded on a Waters ZMD Single Quadrapole Mass Spectrometer. Optical rotations were measured on a Perkin Elmer Polarimeter 341.

**Example 1: Synthesis of two mixtures each containing 4 stereoisomers of methyl esters of AH13205**

*(a) (i) Synthesis of 1- (4-bromophenyl) hexan-1-one (1)*

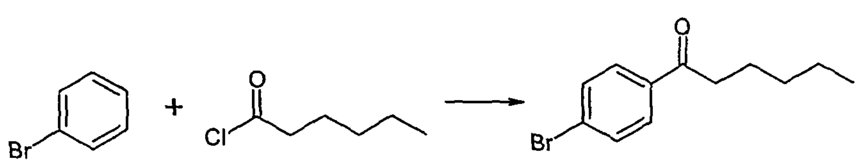

1

To a stirred ice-cooled mixture of $AlCl_3$ (83 g) and bromobenzene (200 mL) under nitrogen was added dropwise hexanoylchloride (75 mL) over a period of 30 minutes. The mixture was then heated to 80°C (external) for 1.5 hours, after which time the solution had turned a deep red. The mixture was then allowed to cool before being poured into 600mL ice water and then extracted with DCM (800 mL). The organic extracts were then washed with brine, dried ($MgSO_4$) and concentrated *in vacuo*. The concentrate was then treated with iso-hexane (1 L) and left in the freezer

overnight, wherein crystallization took place. The slightly off-white solid was filtered and washed with more cold hexane, to yield the title compound (84g). Shown to be adequately pure by nmr and tlc. $^1H$ NMR ($CDCl_3$, δ): 0.95 (3H, t); 1.4 (4H, m); 1.75 (2H, m); 2.95 (2H, t); 7.6 (2h, d); 7.85 (2H, d)

(a) (ii) *Synthesis of (S) -1- (4-Bromophenyl) hexan-1-ol (S-BPH) (**2a**)*

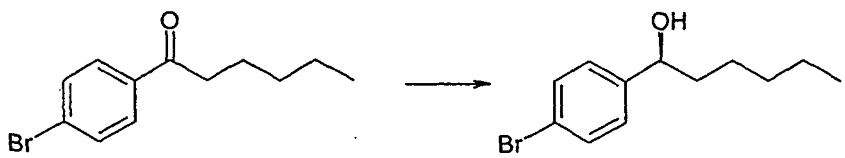

1 2a

To a solution of (-)-DIP chloride [B-chlorodiisopinocampheylborane] (13.5g) in anhydrous THF (20ml), cooled to -25°C, was added a solution of 1-(4-bromophenyl)hexan-1-one (10g) in anhydrous THF (20ml) over 5-10 minutes keeping the temperature below -20°C. The mixture was kept at -25°C for the next 6 hours then added to a vigorously stirred mixture of diethanolamine (12ml) and triethylamine (10ml) in ether (250ml). The mixture was left stirring overnight, washed with dilute hydrochloric acid, brine, dried over sodium sulphate and evaporated *in vacuo.* Compound **2a** (4.5g; m.p. 70-71°C) was obtained following silica-gel column chromatography of the residue in dichloromethane followed by re-crystallisation from heptane.

$[\alpha]_D^{24} = -26.5$ (c = 4.00; $CHCl_3$)

$^1H$ NMR ($CDCl_3$, δ) : 0.85 (3H, t); 1.2-1.9 (9H, m); 4.6 (1H, t); 7.15 (2H, d); 7.45 (2H, d).

HPLC (Chiracel OD 250 x 4.6mm, eluant hexane:IPA 99:1, flow rate 0.5ml/min, λ=254nm): 44 minutes, e.e.100%.

(a) *(iii) Synthesis of (R) -1- (4-Bromophenyl) hexan-1-ol (R-BPH) (**2b**)*

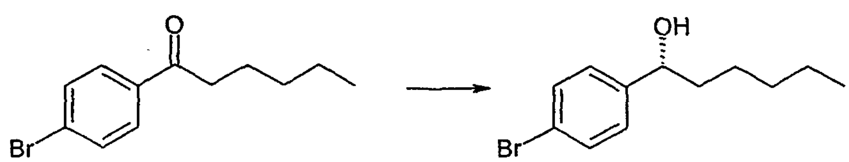

1 2b

Compound **2b** (4g; m.p. 70-71°C) was made from (+)-DIP chloride [B-chlorodiisopinocampheylborane] (13.5g) and 1-(4-bromophenyl)hexan-1-one (10g) by an analogous method to that described in Example 1(a)(ii).

$[\alpha]_D^{24} = +27.3$ (c= 4.06; $CHCl_3$)

m/z (EIMS) : 256, 258

$^1H$ NMR ($CDCl_3$, δ) : 0.85 (3H, t); 1.2-1.9 (9H, m); 4.6 (1H, t); 7.15 (2H, d); 7.45 (2H, d).

HPLC (Chiracel OD 250 x 4.6mm, eluant hexane: IPA 99:1, flow rate 0.5ml/min, λ=254nm): 47 minutes, e.e.100%.

The absolute stereochemistry of compounds **2a** and **2b** was assigned by analogy with a literature method for reducing long chain aromatic ketones described by Brown, H.C., et al. J. Am. Chem. Soc., 110, 1539-1546 (1998). The alcohols were shown to be essentially homochiral by chiral HPLC.

*(a) (iv) Alternative synthesis of (S)- (-) -1- (4-bromphenyl) hexan-1-ol (S-BPH) (**2a**)*

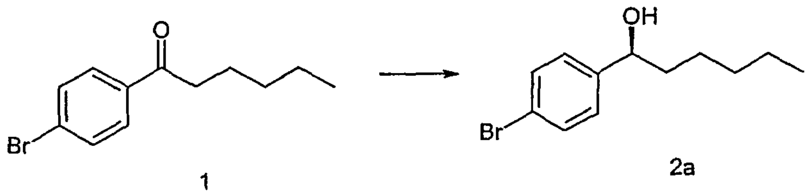

1

2a

$BH_3$.THF (1 M in THF, 234 mL, 234 mmol) was stirred under nitrogen and cooled to -10°C before being treated with (R)-2-methyl-CBS-oxazaborolidine (24 mL, 24 mmol). After being left stirring for 20 minutes, 1-(4-bromophenyl)hexan-1-one (1) (48.3 g) was added as a solution in THF (379 mL) over a period of 1 hour, and thereafter left for a further 20 minutes before quenching carefully with MeOH (100 mL) - $H_2$ evolves. Advisable to perform the quench at RT since MeOH reacts slowly at -10°C. The mixture was then concentrated *in vacuo,* then redissolved in MeOH (300 mL) and treated with HCl (2 M in $Et_2O$, 40 mL). The solution was stirred for 5 minutes before concentrating *in vacuo,* triturating with $Et_2O$ and removing the solid by filtration. The mother liquors were again concentrated *in vacuo* then recrystallized from hexane (480 mL, 10 vol.) at -10°C to yield the title compound as a fluffy white solid (20.7 g).

(b) (i) *Synthesis of (S)-1-(4-Bromophenyl) -1- (tert-butyldimethylsilyloxy)hexane (**3a**)*

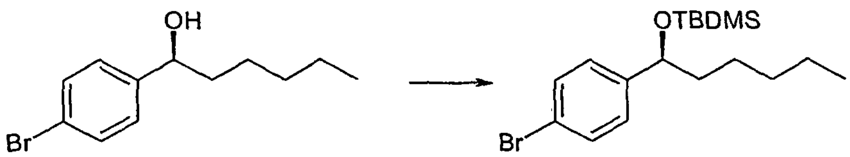

2a

3a

A mixture of S-BPH (**2a**) (10g), tert-butyldimethylsilyl chloride (7g) and imidazole (3.7g) were stirred in anhydrous dimethylformamide (100ml) for 16 hours. The mixture was partitioned between petroleum ether and water and the layers separated. The organic layer was washed with water, brine, dried over sodium sulphate and evaporated *in vacuo.* Compound **3a** (14.5g) was obtained as an oil following column chromatography of the residue in petroleum ether.
m/z (EIMS) : 370, 372
$^1$H NMR ($CDCl_3$, $\delta$): -0.2 (3H, s); 0.0 (3H, s); 0.85 (9H, s); 0.85 (3H, t); 1.25 (6H, m) 1.55 (2H, m); 4.6 (1H, m); 7.1 (2H, d); 7.4 (2H, d).

*(b) (ii) Synthesis of (R)-1-(4-Bromophenyl)-1-(tert-butyldimethylsilyloxy)hexane (3b)*

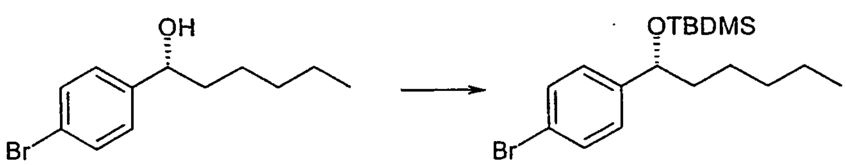

2b

3b

Compound **3b** (18.5g) was made from R-BPH (**2b**) (12.5g) by an analogous method to that described in Example 1(b)(i).
m/z (EIMS): 370, 372
$^1$H NMR ($CDCl_3$, $\delta$) : -0.2 (3H, s) ; 0.0 (3H, s); 0.85 (9H, s); 0.85 (3H, t); 1.25 (6H, m) 1.55 (2H, m); 4.6 (1H, m); 7.1 (2H, d); 7.4 (2H, d).

*(c) Synthesis of 2-(6-carbomethoxyhexyl)cyclopent-2-en-1-one (5)*

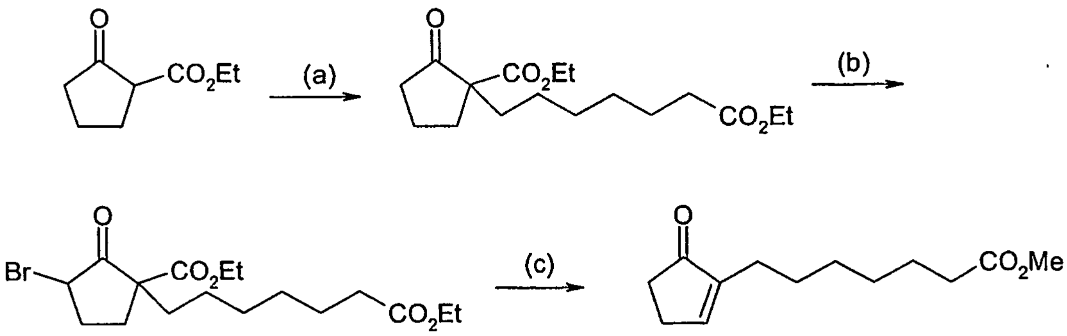

5

(a) ethyl 7-bromoheptanoate, sodium hydride
(b) bromine
(c) (i) acid; (ii) esterify

This known compound, which is commercially available, was prepared in three steps from ethyl 2-oxocyclopentane carboxylate by the methods of Bagli, J. et al., J. Org. Chem., 1972, 37, 2132-2138 and Bernady, K.F., J. Org. Chem., 1980 45, 4702-4715.

*(d) (i) Synthesis of 2-{4-[1-(R)-(tert-butyldimethylsilyloxy)hexyl]phenyl}-5-oxo-cyclopentaneheptanoic acid, methyl ester diastereomers (circa* 3:1 *trans: cis mixture)) (**6b**)*

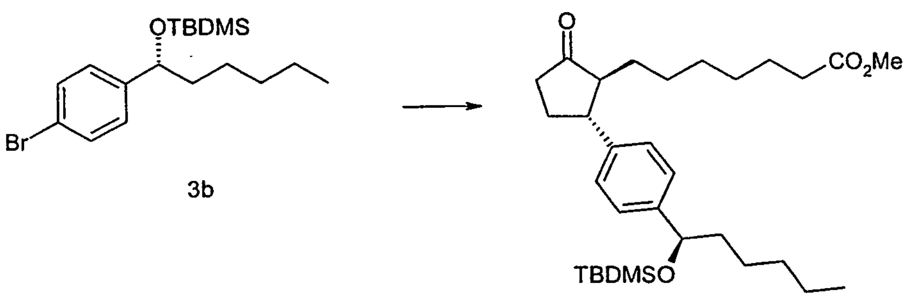

3b

& its diastereoisomer
+ ~30% *cis*-isomer (6b)

To a solution of (R)-1-(4-bromophenyl)-1-(tert-butyldimethylsilyloxy)hexane (**3b**) (7.2g) in anhydrous diethyl ether (100ml) was added tert-butyllithium (1.5M in hexanes; 28ml) dropwise at -78°C, not allowing the temperature to rise above -60°C. The mixture was left at -78°C for a further 3 hours. A slurry of copper (1) pentyne (2.5g) in anhydrous diethyl ether (56ml) was treated with hexamethylphosphorous triamide (8ml) and the mixture stirred at room temperature for several minutes to form a solution. This freshly prepared solution was now added dropwise to the aryllithium solution at -78°C and left for a further hour at -78°C, whereupon a solution of 2-(6-carbomethoxyhexyl)cyclopent-2-en-1-one (**5**) (4g) in anhydrous diethyl ether (40ml) was added. The reaction mixture was held at -78°C for 15 minutes then at -25°C to -10°C for a further hour. The cold mixture was partitioned quickly between dilute hydrochloric acid and ether, the organic layer separated, washed with brine, dried over sodium sulphate and evaporated *in vacuo.* Compounds **6b** (7.2g) were obtained as a *circa* 3:1 mixture of *trans: cis* isomers following silica-gel column chromatography of the residue in 2:1 dichloromethane:

petroleum ether then 3:17 ethyl acetate:petroleum ether.
$^1$H NMR ($CDCl_3$, $\delta$) - *trans*-diastereomers: -0.25 (3H, s); 0.0 (3H, s); 0.85 (9H, s); 0.8-2.0 (22H, m); 2.2-2.6 (6H, m); 2.95 (1H, m); 3.65 (3H, s); 4.6 (1H, m); 7.15 (2H, d); 7.25 (2H, d).
$^1$H NMR ($CDCl_3$, $\delta$) - *cis*-diastereomers: -0.27 (3H, s); -0.02 (3H, s); 0.85 (9H, s); 0.8-2.0 (22H, m); 2.2-2.6 (6H, m); 3.55 (1H, m); 3.65 (3H, s); 4.6 (1H, m); 7.0 (2H, d); 7.15 (2H, d).

*(d) (ii) 2-{4-[1-(S)-(tert-butyldimethylsilyloxy)hexyl]phenyl}-5-oxo-cyclopentaneheptanoic acid, methyl ester diastereomers (circa 3:1 trans: cis mixture) (**6a**)*

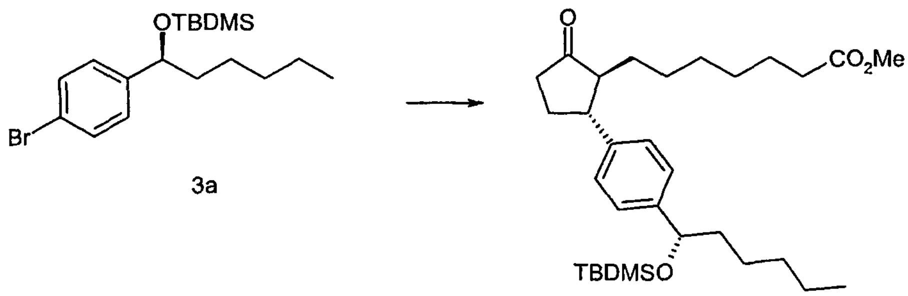

3a

& its diastereoisomer
+ ~30% *cis*-isomer (6a)

The title compound and its diastereoisomer, and about 30% of their *cis*-isomers (**6a**), (7.3g) were made from (*S*) -1- (4-bromophenyl)-1-(*tert*-butyldimethylsilyloxy)hexane (**3a**) (7.2g) and 2-(6-carbomethoxyhexyl)cyclopent-2-en-1-one (5) (4g) by an analogous method to that described in Example 1(d)(i).
$^1$H NMR ($CDCl_3$, $\delta$) - *trans* diastereomers : -0.25 (3H, s); 0.0 (3H, s); 0.85 (9H, s); 0.8-2.0 (22H, m); 2.2-2.6 (6H, m); 2.95 (1H, m); 3.65 (3H, s); 4.6 (1H, m); 7.15 (2H, d); 7.25 (2H, d).
$^1$H NMR ($CDCl_3$, $\delta$) - *cis* diastereomers : -0.27 (3H, s); -0.02 (3H, s); 0.85 (9H, s); 0.8-2.0 (22H, m); 2.2-2.6 (6H, m); 3.55 (1H, m); 3.65 (3H, s); 4.6 (1H, m); 7.0 (2H, d); 7.15 (2H, d).

(d) (iii) *Alternative synthesis of 2-{4-[1-(R)-(tert-butyldimethylsilyloxy)hexyl]phenyl}-5-oxo-cyclopentaneheptanoic acid, methyl ester diastereomers (circa 3:1 trans: cis mixture) (**6b**)*

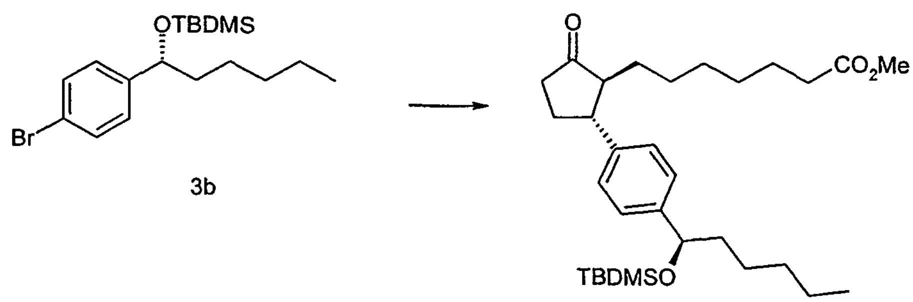

3b

6b & its diastereoisomer
+ ~30% *cis*-isomer

A mixture of (*R*)-1-(4-bromophenyl)-1-(tert-butyldimethylsilyloxy)hexane (**3b**) (0.8g), magnesium turnings (0.11g), a crystal of iodine and 1,2-dibromoethane (5μl) in anhydrous THF (4ml) were boiled to reflux to initiate reaction, then kept

at 35°C for 2 hours to form the Grignard solution. Lithium chloride (0.125g) and copper (1) bromide.dimethyl sulphide complex (0.61g) were stirred in anhydrous THF (4.5ml) for a few minutes then cooled to -78°C whereupon the Grignard solution was added dropwise. The resulting mixture was left for 5 minutes at -78°C then trimethylsilyl chloride (0.38ml) was added followed by a solution of 2-(6-carbomethoxyhexyl)cyclopent-2-en-1-one (0.18g) in anhydrous THF (1.5ml). The mixture was kept at - 78°C for 15 minutes, at 0°C for 30 minutes then allowed to warm up to room temperature for an hour. The mixture was recooled to -20°C whereupon dilute hydrochloric acid (4ml) was added and the mixture stirred vigorously for two minutes. The cold mixture was partitioned between petroleum ether and saturated ammonium chloride solution and the layers separated. The organic layer was washed with brine, dried over sodium sulphate and evaporated *in vacuo.* Compounds 6b (0.20g) were isolated as a mixture of cis and *trans* isomers following silica-gel column chromatography of the residue in 4:1 petroleum ether:ethyl acetate.

$^1H$ NMR ($CDCl_3$, δ) - *trans* diastereomers : -0.25 (3H, s); 0.0 (3H, s); 0.85 (9H, s); 0.8-2.0 (22H, m); 2.2-2.6 (6H, m); 2.95 (1H, m); 3.65 (3H, s); 4.6 (1H, m); 7.15 (2H, d); 7.25 (2H, d).

$^1H$ NMR ($CDCl_3$ δ) - *cis* diastereomers: -0.27 (3H, s); -0.02 (3H, s); 0.85 (9H, s); 0.8-2.0 (22H, m); 2.2-2.6 (6H, m); 3.55 (1H, m); 3.65 (3H, s); 4.6 (1H, m); 7.0 (2H, d); 7.15 (2H, d).

(e) (i) *Synthesis of trans and cis-2-[4-(1-(S)-hydroxyhexyl)phenyl]-5-oxo-cyclopentaneheptanoic* acid, methyl ester *diastereomers* (***7a***) (***Mixture 1***)

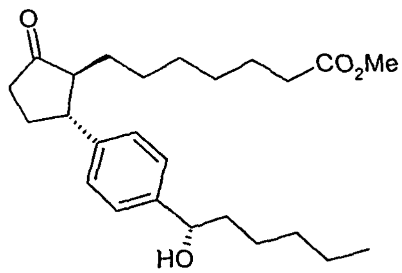

A

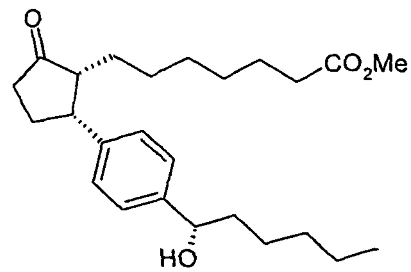

B

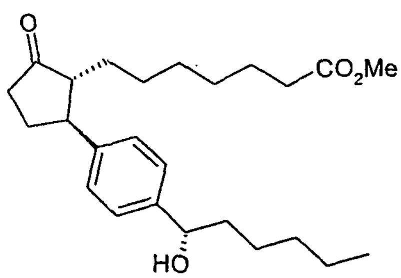

C

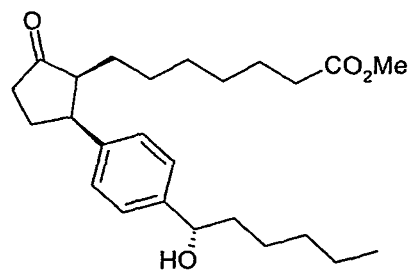

D

7a (MIXTURE 1)

A : (1S,2R)-2-[4-(1-(S)-hydroxyhexyl)phenyl]-5-oxo-cyclopentaneheptanoic acid, methyl ester [SRS]
B: (1R,2R)-2-[4-(1-(S)-hydroxyhexyl)phenyl]-5-oxo-cyclopentaneheptanoic acid, methyl ester [RRS]
C: (1R,25)-2-[4-(1-(S)-hydroxyhexyl) phenyl]-5-oxo-cyclopentaneheptanoic acid, methyl ester [RSS]
D: (1S,2S)-2-[4-(1-(S)-hydroxyhexyl)phenyl]-5-oxo-cyclopentaneheptanoic acid, methyl ester [SSS]

2-{4-[1-(S)-(tert-butyldimethylsilyloxy)hexyl]phenyl}-5-oxo-cyclopentaneheptanoic acid, methyl ester diastereomers (**6a**) (0.2g; of *circa* 3:1 *trans:cis* composition) was stirred in a mixture of THF (3.5ml) and dilute hydrochloric acid (2M; 1ml) for 20 hours at 25°C. The reaction mixture was added to brine and extracted twice with dichloromethane. The combined organic layers were dried over sodium sulphate and evaporated *in vacuo.* 7a (Mixture 1) (0.095g) was obtained as an

oil (of *circa* 95:5 *trans:cis* composition) following silica-gel column chromatography of the residue in 3:1 petroleum ether: ethyl acetate then 200:3 dichloromethane:methanol.
m/z (EIMS) : 402
$^1$H NMR ($CDCl_3$, $\delta$) - *trans* diastereomers only: 0.8-2.0 (23H, m); 2.2-2.6 (6H, m); 2.95 (1H, m); 3.65 (3H, s); 4.65 (1H, t) ; 7.25 (2H, d) ; 7.35 (2H, d).

(e) (ii) *trans and cis-2-[4-(1-(R)-hydroxyhexyl)phenyl]-5-oxo-cyclopentaneheptanoic acid, methyl ester diastereomers* (***7b***) *(Mixture 2)*

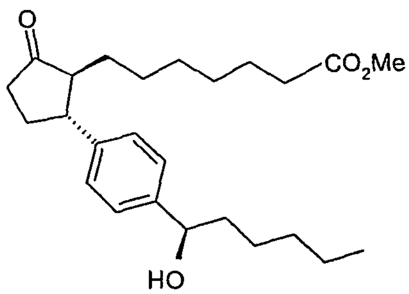

E

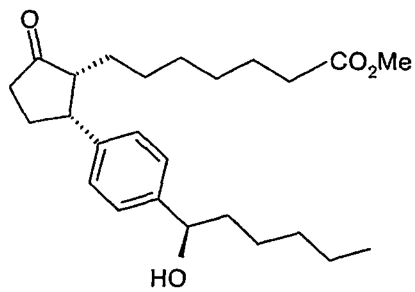

F

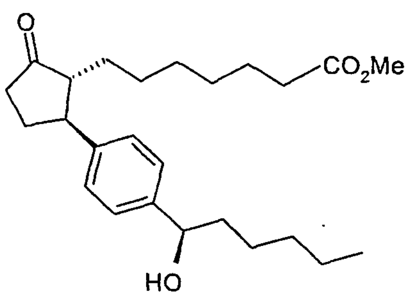

G

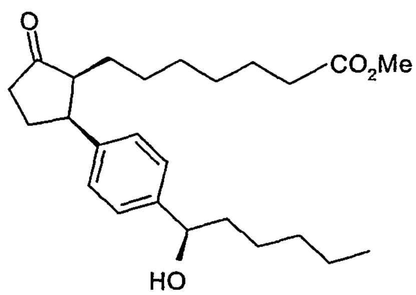

H

7b (MIXTURE 2)

E: (1S,2R)-2-[4-(1-(R)-hydroxyhexyl) phenyl]-5-oxo-cyclopentaneheptanoic acid, methyl ester [SRR]
F: (1R,2R)-2-[4-(1-(R)-hydroxyhexyl)phenyl]-5-oxo-cyclopentaneheptanoic acid, methyl ester [RRR]
G: (1R,2S)-2-[4-(1-(R)-hydroxyhexyl)phenyl]-5-oxo-cyclopentaneheptanoic acid, methyl ester [RSR]
H: (1S,2S)-2-[4-(1-(R)-hydroxyhexyl)phenyl]-5-oxo-cyclopentaneheptanoic acid, methyl ester [SSR]
Compounds **7b** (0.15g; of *circa* 95:5 *trans:cis* composition)) were made from 2-{4-[1-(R)-(tert-butyldimethylsilyloxy)hexyl] phenyl}-5-oxo-cyclopentaneheptanoic acid, methyl ester diastereomers (0.3g; of *circa* 3:1 *trans:cis* composition)(**6b**) by an analogous method to that described in Example 1(e)(i).
m/z (EIMS): 402
$^1$H NMR ($CDCl_3$, $\delta$) - *trans* isomer only: 0.8-2.0 (23H, m); 2.2-2.6 (6H, m); 2.95 (1H, m); 3.65 (3H, s); 4.65 (1H, t); 7.25 (2H, d); 7.35 (2H, d).

**Example 2: Separation of trans-2-[4-(1-hydroxyhexyl)phenyl]-5-oxo-cyclopentaneheptanoic acid methyl ester diastereomers**

HPLC of a 90 mg sample of either ester mixture 1 or 2 on a chiral stationary phase (ChiralPak AD, Daicel Chemical Industries, Japan) using a mobile phase of 100% ethanol afforded complete separation on a column of 25 cm in length by 2 cm internal diameter, in about an hour. A 1g sample of either mixture was separated in eleven consecutive

90 mg runs (flow rate 4ml/min; detection 230 nm). The recovered esters were then hydrolysed to the acids as follows. Methyl ester (0.45g) in 4:1 v/v tetrahydrofuran in water (40ml) was treated with 1M lithium hydroxide in water (1.37 ml, 1.2equiv.) added dropwise and the solution was stirred overnight at ambient temperature. The solution was concentrated in vacuo, diluted with water, acidified to pH~1 and extracted into ethyl acetate. The extract was dried over magnesium sulphate, filtered and concentrated in vacuo at 30˚C to give the acid as an oil. The recoveries of acids starting from 1g of each mixture of methyl esters were: from Peak 1, mixture 1: 0.392g; from Peak 2, mixture 1: 0.427g; from Peak 1, mixture 2: 0.433g and from Peak 2, mixture 2: 0.381g.

Optical rotations were recorded for the acids derived from each of the methyl ester peaks indicated. The acids obtained from the isolated peaks are hereinafter referred to as Peak m, Mixture m acid, for clarity

Peak 1, mixture 1 acid
$[\alpha]^{25}$ -422.4 (365nm), -142.9 (436nm), -57.3 (546nm), -47.9 (578nm), -45.1 (589nm)
(c= 0.6275, $CHCl_3$; path length 100mm)

Peak 2, mixture 1 acid
$[\alpha]^{25}$ +314.9 (365nm), +80.6 (436nm), +22.7 (546nm), +17.3 (578nm), +16.0 (589nm) (c= 1.365, $CHCl_3$; path length 100mm)

Peak 1, mixture 2 acid
$[\alpha]^{25}$-299.4 (365nm), -77.8 (436nm), -22 .2 (546nm), -16.9 (578nm), -15.5 (589nm)
(c= 0.81, $CHCl_3$; path length 100mm)

Peak 2, mixture 2 acid
$[\alpha]^{25}$ +422.6 (365nm), +140.6 (436nm), +55.5 (546nm), +45.9 (578nm), +42.9 (589nm)
(c= 0.885, $CHCl_3$; path length 100mm)

Chemical purity was determined by NMR and LC-MS; chiral purity was determined by chiral HPLC as described below. $^1H$ NMR ($CDCl_3$) confirmed the structure of the acid and showed the presence of only a trace of the methyl ester; a low level impurity, probably the cis- isomer, was always present together with residual ethyl acetate.

$^{13}C$ NMR showed very small differences between diastereomers of the esters. Peak 1, mixture 1 acid and peak 2, mixture 2 acid were shown to be enantiomers, as were peak 2, mixture 1 acid and peak 1, mixture 2 acid. This correlates with the optical rotation data for each compound shown above.

**Example 3: Hydrolysis of Separated Methyl Esters**

0.45g of a methyl ester (as separated in Example 2) was dissolved in 40ml of a 4:1 v/v solution of THF in water; 1.37ml of 1M lithium hydroxide solution (1.2equiv.) was added dropwise, and the solution then stirred overnight at ambient temperature. The reaction was then examined by LC-MS, which typically showed clean formation of the free acid, with only a trace of ester remaining. The reaction was concentrated down under vacuum to remove THF, and more water added; the stirred solution was treated dropwise with 1M hydrochloric acid to give pH~1, and the solution then equilibrated with ethyl acetate; the aqueous layer was removed, and the ethyl acetate layer washed with brine, dried over magnesium sulphate, filtered, and evaporated under vacuum. The residual oil was transferred to a weighed vial in a little ethyl acetate, and solvent removed under a stream of nitrogen; the sample was then placed in a drying pistol and pumped on overnight at 30˚C/1mbar.

$^1H$ NMR ($CDCl_3$) confirmed the structure of the product as the free acid, and typically showed the presence of only a trace of methyl ester; a low level impurity, thought to be the cis-isomer, was always present, as was residual ethyl acetate.

The chiral purity of the product was assessed by re-esterifying a small sample of each of the four separated acid isomers and then analysing the esters by analytical chiral HPLC. About 5 mg of acid was dissolved in ether and treated with a freshly prepared solution of diazomethane in ether, to give a permanent yellow colour. After standing for 30 minutes at ambient temperature the solution was blown to dryness under nitrogen and re-dissolved in ethanol for chiral HPLC. The conditions used for the analysis were; analytical ChiralPak AD column (25 cm by 0.46 cm), 100% ethanol as stationary phase, flow rate of 0.25 ml/min UV detection (230 nm) at ambient temperature. Typical retention times for each isomer were: Peak 1, mixture 1 acid: 23.5 min; Peak 2, mixture 1 acid: 56 min; Peak 1, mixture 2 acid: 23.7 min; Peak 2, mixture 2 acid: 35 min. The chiral purity of each sample was essentially 100%.

**Example 4: Assignment of the absolute stereochemistry of four stereoisomers of *trans*-2-[4-(1-hydroxyhexyl) phenyl]-5-oxo-cyclopentaneheptanoic acid**

The absolute stereochemistry of AH13205 stereoisomers was determined by assigning the absolute configuration of the acid side chain-cyclopentanone junction using circular dichroism. The four stereoisomers of AH-13205, which all have the trans-configuration of the two side chains on the cyclopentanone have been separated (peak 1, mixture 1 acid; peak 2, mixture 1 acid; peak 1, mixture 2 acid and peak 2, mixture 2 acid); these are oils at room temperature.

To aid in assigning the absolute configuration, standards were also analysed. These are prostaglandin $E_2$ ($PGE_2$), (S)-1-(4-bromophenyl)hexan-1-ol (S-BPH) and (R)-1-(4-bromophenyl)hexan-1-ol (R-BPH) (shown below).

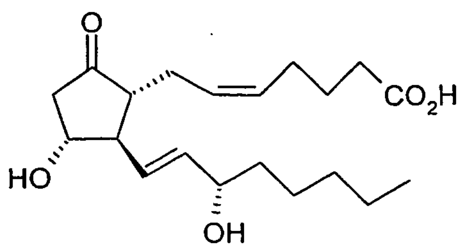

prostaglandin $E_2$ ($PGE_2$)

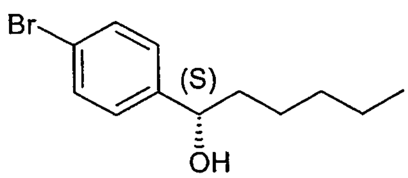

(S)-1-(4-bromophenyl)hexan-1-ol (S-BPH)

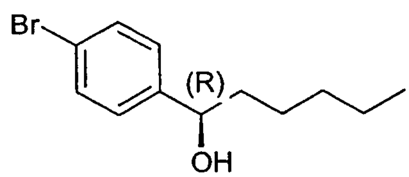

(R)-1-(4-bromophenyl)hexan-1-ol (R-BPH)

The samples were stored at room temperature and were dissolved in 100% ethanol and diluted to the concentrations as shown in table 1 prior to analysis.

Table 1

| **Sample** | *Mass* | *Final concentration* |
|---|---|---|
| Peak 1, mixture 1 acid | 15.7 mg | 19 mg/mL |
| Peak 2, mixture 1 acid | 10.6 mg | 19 mg/mL |
| Peak 1, mixture 2 acid | 7.1 mg | 19 mg/mL |
| Peak 2, mixture 2 acid | 28.5 mg | 19 mg/mL |
| $PGE_2$ | Approximately 5 mg | 0.7 mg/mL |
| R-BPH | 15 mg | 0.7 mg/mL |
| S-BPH | 20 mg | 0.7 mg/mL |

A 1.0 cm pathlength quartz cuvette was used for the analysis of $PGE_2$, R-BPH and S-BPH. A 0.1 cm pathlength quartz cuvette was used for the analysis of the four AH13205 stereoisomers. Ethanol volumes were measured with a Gilson micropipette, for which they are in calibration if used quickly.

Instrument Calibration and Acceptance Criteria

The wavelength accuracy calibration criteria were met. Analysis of 0.06% (w/v) aqueous ammonium d-10-camphor sulfonate (ACS) showed signal intensity within specification, therefore no scaling factors were necessary.

The corresponding water baseline was subtracted from each sample spectrum, and the spectra were zeroed in the 360 - 400 nm region, which was outside the absorption band.

Results

Single analyses of UV and CD were undertaken for all samples. The UV and CD baseline subtracted and zeroed data for the standards $PGE_2$ and R-/S-BPH are shown in Figures 1a, 1b, 2a and 2b. The data for the four stereoisomers of AH13205 is shown in Figure 3a and 3b. The results are summarised in Table 2.

Table 2

| *Sample* | *Final concentration* | *Pathlength* | *First UV band/nm* | *UV intensity maximum* | *First CD band/ nm* | *CD intensity maximum* |
|---|---|---|---|---|---|---|
| **Peak 1 Mixture 1 acid** | 19 mg/mL | 0.1 cm | 263.5/287.0 | 1.42/0.28 | 297.0 | -630.5 |
| **Peak 2 Mixture 1 acid** | 19 mg/mL | 0.1 cm | 263.5/287.0 (sh) | 1.17/0.23 | 297.0 | 511.5 |
| **Peak 1 Mixture 2 acid** | 19 mg/mL | 0.1 cm | 263.5/287.0 | 1.59/0.33 | 297.0 | -685.0 |
| **Peak 2 Mixture 2 acid** | 19 mg/mL | 0.1 cm | 263.5/287.0 | 1.34/0.28 | 297,0 | 583.5 |
| **$PGE_2$** | 0.7 mg/mL | 1 cm | 284.5 | 0.13 | 299 | -239 |
| **R-BPH** | 0.7 mg/mL | 1 cm | 267.0 | 0.74 | 269 | -5.4 |
| **S-BPH** | 0.7 mg/mL | 1 cm | 267.0 | 0.76 | 269 | 6.46 |

The stereoisomeric samples (peak 1, mixture 1 acid; peak 2, mixture 1 acid; peak 1, mixture 2 acid and peak 2, mixture 2 acid) have two or three transitions of interest, one due to the $n \rightarrow \pi^*$ transition at ~290 nm and one due to the aromatic ring $\pi \rightarrow \pi^*$ transition at about 260 nm and the next transition below this region. $PGE_2$ was used as a model for the $n \rightarrow \pi^*$ part of the molecule and R-/S-BPH to model the $\pi \rightarrow \pi^*$ part.
The $\pi \rightarrow \pi^*$ absorbances were found to be weak and did not interfere with the analysis of the $n \rightarrow \pi^*$ band.

The induced CD for $PGE_2$ is expected to be dominated by the acid group chain $\alpha$- to the carbonyl. Analysis using the standard octant rule shows that this chain lies in the -z, +y and -x octant thus making *-xyz* (and therefore the expected CD curve) negative. This was observed experimentally (Table 2). Peak 1, mixture 1 acid and peak 1, mixture 2 acid samples showed negative CD curves and therefore have the same absolute stereochemistry at the side chain junction as $PGE_2$. Peak 2, mixture 1 acid and peak 2, mixture 2 acid samples showed positive CD curves and therefore have the opposite absolute stereochemistry from $PGE_2$ at this junction.

Both by pattern-matching with $PGE_2$ and with the octant rule correlation, the peak 1, mixture 1 acid and peak 1, mixture 2 acid samples have been shown to have the same absolute configuration as $PGE_2$ at the carbon $\alpha$- to the carbonyl which is the point of attachment of the acid side chain to the cyclopentanone. The peak 2, mixture 1 acid and peak 2, mixture 2 acid samples have the opposite configuration to $PGE_2$ at this point. These results are shown in table 3.

Table 3

| | Stereoisomer |
|---|---|
| Peak 1, Mixture 1 acid | (1) or (3) |
| Peak 2, Mixture 1 acid | (2) or (4) |

| | |
|---|---|
| Peak 1, Mixture 2 acid | (1) or (3) |
| Peak 2, Mixture 2 acid | (2) or (4) |

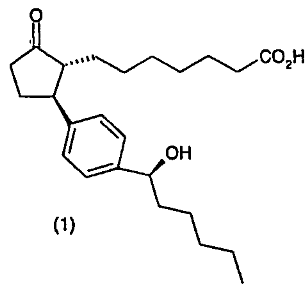
(1)

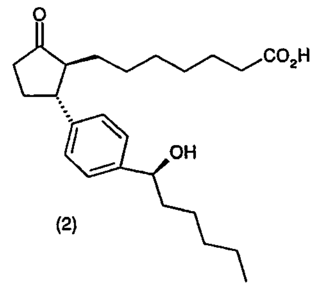
(2)

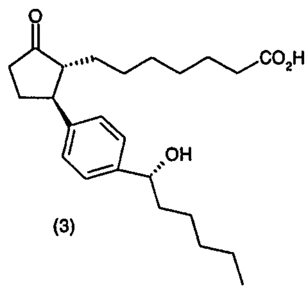
(3)

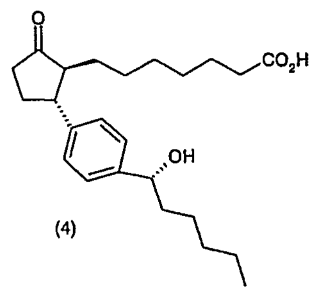
(4)

The determination of absolute stereochemistry by these CD studies is in agreement with the expected results of the stereoselective synthesis described for compound 17 [RSS], as predicted by comparison with the results from the literature of related stereoselective syntheses using compound (22).

As the stereochemistry at the 1- position of the hexyl chain in the 4-(1-hydroxyhexyl)phenyl side chain is known from the starting materials, the assignements in table 3 allow the stereochemistry of each of the isomers to be determined. These results are shown below.

AH13205 *trans*- Stereoisomers

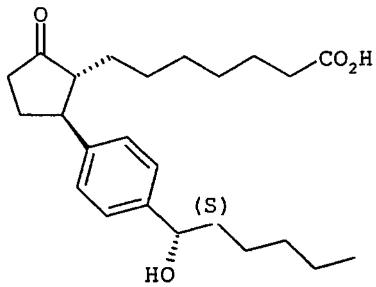

Peak 1, mixture 1 acid (C)
(1R,2S)-2-[4-(1-(S)-hydroxyhexyl)phenyl]-5-oxo-cyclopentaneheptanoic acid

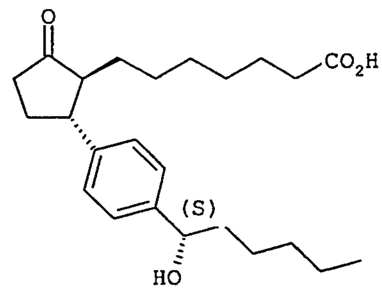

Peak 2, mixture 1 acid (A)
(1S,2R)-2-[4-(1-(S)-hydroxyhexyl)phenyl]-5-oxo-cyclopentaneheptanoic acid

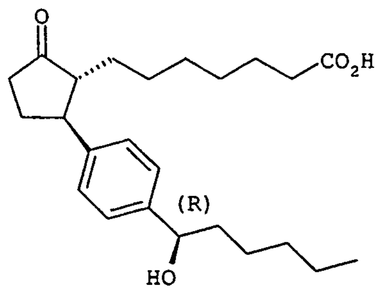

Peak 1, mixture 2 acid (G)
(1R,2S)-2-[4-(1-(R)-hydroxyhexyl)phenyl]-5-oxo-cyclopentaneheptanoic acid

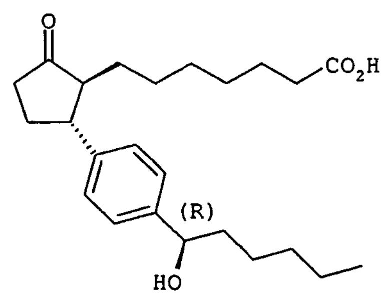

Peak 2, mixture 2 acid (E)
(1S,2R)-2-[4-(1-(R)-hydroxyhexyl)phenyl]-5-oxo-cyclopentaneheptanoic acid

**Example 5: Stereoselective preparation of (1R,2S)-2-[4-(1-(S)-hydroxyhexyl)phenyl]-5-oxo-cyclopentaneheptanoic acid (peak 1, mixture 1 acid).**

(a) Preparation of (1R, 2S)-2-{4-[1-(S)-tert-butyldimethylsilyloxy)hexyl]-phenyl}-5-oxo-cyclopentanecarboxylic acid, (1R,2S,3R,4S)-{3-N-benzenesulfonyl-N-(3,5-dimethylphenyl)amino]-2-bornyl} ester (25)

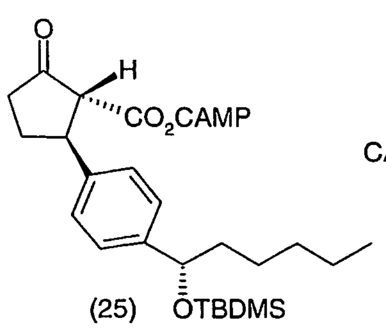

(25)

CAMP =

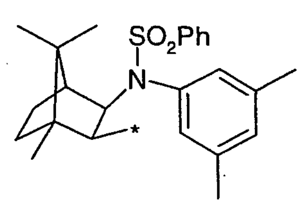

To a solution of (S)-1-(4-bromophenyl)-1-(tert-butyldimethyl-silyloxy)hexane (1.8g) in anhydrous THF (24ml) was added at -78°C tert-butyllithium (1.7M in pentane; 5.6ml) keeping the temperature below -65°C. After stirring for 2 hours at

-78°C, lithium-2-thienylcyanocuprate (0.25M in THF; 19.2ml) was added and the mixture left at -78°C for an hour. A solution of 5-oxo-cyclopentenecarboxylic acid, (1R,2S,3R,4S)-{3-[N-benzenesulfonyl-N-(3,5-dimethylphenyl)amino]-2-bornyl} ester (1.7g) in anhydrous THF (15ml) was then added and the resulting mixture left at -78°C for 75 minutes. Saturated ammonium chloride solution (15ml) was then added and the mixture allowed to warm up to 10-15°C over the next hour. Further saturated ammonium chloride solution was added and the mixture extracted twice with ethyl acetate. The combined organic extracts were washed with ammonium chloride solution and subsequently with brine then dried over sodium sulphate and evaporated *in vacuo.* (1R,2S)-2-{4-[1-(S)-(tert-butyldimethylsilyloxy)hexyl]phenyl}-5-oxo-cyclopentanecarboxylic acid, (1R, 2S, 3R, 4S) -{ 3- [N-benzenesulfonyl-N-(3,5 -dimethylphenyl)amino]-2-bornyl} ester (1.8g) was obtained as a foam following silica-gel column chromatography of the residue in 3:1 petroleum ether:diethyl ether.

$^1$H NMR ($CDCl_3$, δ) : (a mixture of keto and enol forms) -0.3, -0.15, -0.1, 0.0, 0.1, 0.35, 0.45, 0.55 (8s); 0.75-2.8 (c); 3.7 (m), 4 .1 (m); 4.55 (m); 5.25 (dd); 5.5-5.8 (2 br s); 6.8(d); 6.95-7.5 (c); 11.0 (enol proton, s).

Mass Spectrum (m/z) $ES^+$: 836.6 $(M+Na)^+$

(b) Preparation of (1R, 2S)-2-{9-[1-(S)-tert-butyldimethylsilyloxy)hexyl]phenyl}-5-oxo-cyclopentanecarboxylic acid, methyl ester (26)

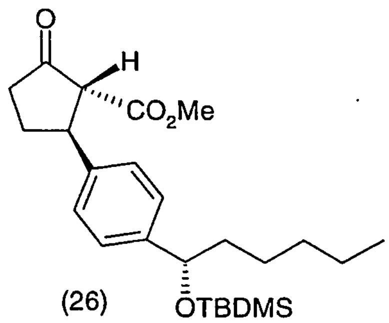

(26)

A solution of (1R, 2S)-2-{4-[1-(S)-(tert-butyldimethylsilyloxy)hexyl]phenyl}-5-oxo-cyclopentanecarboxylic acid, (1R,2S, 3R,4S)-{3-[N-benzenesulfonyl-N-(3,5-dimethylphenyl)amino]-2-bornyl} ester (1.1g) in anhydrous methanol (60ml) was heated to 150°C in a sealed tube for 3 hours. After evaporation of the solvent *in vacuo*, (1R,2S)-2-{4-[1-(S)-(tert-butyldimethylsilyloxy)hexyl]phenyl}-5-oxo-cyclopentanecarboxylic acid, methyl ester (390mg) was obtained as an oil following silica-gel column chromatography of the residue in 2:1 dichloromethane:petroleum ether.

$^1$H NMR ($CDCl_3$, δ): keto form -0.2 (3H, s) ; 0.0 (3H, s) ; 0.85 (9H, s); 0.85 (3H, t); 1.1-1.7 (8H, c); 1.95 (1H, c); 2.5 (3H, c); 3.35 (1H, d); 3.7 (3H, s); 3.8 (1H, m); 4.6 (1H, t); 7.15 (2H, d) ; 7.25 (2H, d).

Mass Spectrum (m/z) $ES^+$: 611.6 $(M+Na)^+$

(c) Preparation of (2S)-1-methoxycarbonyl-2-{4-[1-(S)-(tert-butyldimethylsilyloxy)hexyl]phenyl}-5-oxo-cyclopentaneheptanoic acid, ethyl ester (27)

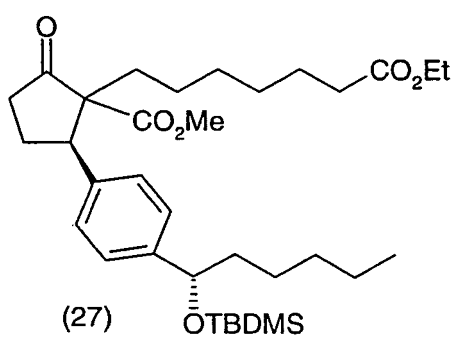

(27)

To a solution of (1R,2S)-2-{4-[1-(S)-(tert-butyldimethylsilyloxy)hexyl]phenyl}-5-oxo-cyclopentanecarboxylic acid, methyl ester (390mg) in anhydrous DME (3.5ml) was added sodium hydride (60% dispersion in oil; 39mg). After leaving for an hour, ethyl 7-bromoheptanoate (0.3ml) and a catalytic amount of sodium iodide were added and the mixture heated to reflux for 20 hours.

After cooling, the mixture was added to ammonium chloride solution and extracted twice with dichloromethane. The combined organic extracts were dried over sodium sulphate and evaporated *in vacuo.* The residue was taken up in anhydrous chloroform (10ml) and a few crystals of para-toluenesulphonic acid monohydrate were added.

After stirring for an hour, sodium bicarbonate solution was added and the organic layer separated, dried over sodium sulphate and evaporated, *in vacuo*. (2S)-1-methoxycarbonyl-2-{4-[1-(S)-(tert-butyldimethylsilyloxy)hexyl]phenyl}-5-oxo-cyclopentaneheptanoic acid, ethyl ester (290mg) was obtained as an oil following silica-gel column chromatography of the residue in 0-5% diethyl ether in dichloromethane.

$^1$H NMR ($CDCl_3$, δ) : -0.2 (3H, s) ; 0.0 (3H, s) ; 0.85 (9H, s); 0.85 (3H, t); 1.1-1.8 (20H, c); 2.0 (1H, c); 2.3 (4H, c); 2.6 (2H, c); 3.4 (3H, s); 3.45 (1H, m); 4.15 (2H, q); 4.6 (1H, t); 7.15 (2H, d) ; 7.25 (2H,d).

Mass Spectrum (m/z) $ES^+$: 455.4 $(M+Na)^+$

<u>(d) Preparation of (1R,2S)-2-{4-[1-(S)-(tert-butyldimethylsilyloxy)hexyl]phenyl}-5-oxo-cyclopentaneheptanoic acid (28).</u>

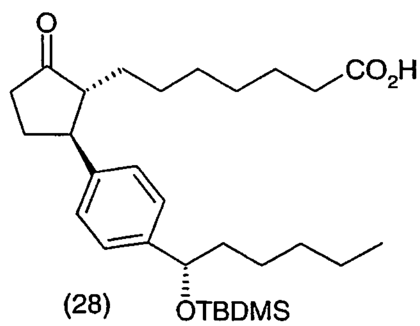

(28)

A mixture of (2S)-1-methoxycarbonyl-2-{4-[1-(S)-(tert-butyldimethylsilyloxy)hexyl]phenyl}-5-oxo-cyclopentaneheptanoic acid, ethyl ester (290mg) and lithium iodide hydrate (650mg) in 2,4,6-collidine (4ml) was heated to reflux for 3 hours.

After cooling the mixture was added to ethyl acetate and washed twice with dilute hydrochloric acid, brine, dried over sodium sulphate and evaporated *in vacuo.* (1R,2S)-2-{4-[1-(S)-(tert-butyldimethylsilyloxy)hexyl]phenyl}-5-oxo-cyclopentaneheptanoic acid (190mg) was obtained as an oil following silica-gel column chromatography of the residue in 4:1 petroleum ether:ethyl acetate.

$^1$H NMR ($CDCl_3$, δ) : -0.2 (3H, s) ; 0.0 (3H, s); 0.85 (9H, s); 0.85 (3H, t); 1.0-2.3 (24H, c); 2.45 (1H, c); 2.9 (1H, m); 4.6 (1H, t); 7.15 (2H, d); 7.25 (2H, d).

Mass Spectrum (m/z) $ES^+$: 525.4 $(M+Na)^+$

<u>(e) Preparation of (1R,2S)-2-[4-(1-(S)-hydroxyhexyl)phenyl]-5-oxo-cyclopentaneheptanoic acid (peak 1, mixture 1 acid).</u>

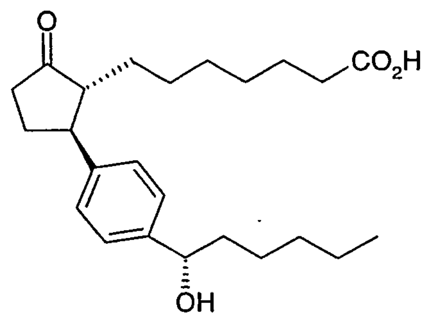

Peak 1, mixture 1 (fig. 7a - C)

A solution of (1R, 2S)-2-{4-[1-(S)-(tert-butyldimethylsilyloxy)hexyl]phenyl}-5-oxo-cyclopentaneheptanoic acid (190mg) in THF (4ml) and 2M hydrochloric acid (1.1ml) was stirred for 20 hours at 30°C. The mixture was added to water and extracted twice with dichloromethane. The combined organic layers were dried over sodium sulphate and evaporated *in vacuo.* (1R,2S)-2-[4-(1-(S)-hydroxyhexyl)phenyl]-5-oxo-cyclopentaneheptanoic acid (115mg) was obtained as an oil following silica-gel column chromatography of the residue in 5:2 petroleum ether:ethyl acetate.
$^1H$ NMR ($CDCl_3$, δ) : 0. 85 (3H, t); 1.0-2.3 (24H, c); 2.45 (1H, c); 2.9 (1H, m); 4.6 (1H, t); 7.25 (2H, d); 7.35 (2H, d).
Mass Spectrum (m/z)$ES^-$: 387.3 $(M-H)^-$
$[\alpha]^{25}$ -416.0 (365nm), -139.5 (436nm), -54.9 (546nm), -45.6 (578nm), -42.4 (589nm) (c=0.51, $CHCl_3$; path length 100mm)

**Example 6: EP binding and agonism**

The ability of compounds to bind to the human $EP_2$ receptor and their selectivity against all other EP receptors can be demonstrated in radioligand competition displacement binding experiments using cell lines stably transfected with the human EP receptors. The ability of compounds to stimulate the $EP_2$ receptor can be demonstrated in a second messenger cAMP functional assay, in primary human lymphocytes, monocytes or in human myometrium.

**Test Details**

*Binding ability to human EP receptors*

Membranes were prepared from cells stably transfected with human EP receptor cDNA. In brief, cells were cultured to confluency, scraped from culture flasks, and centrifuged (800 g, 8 minutes, 4°C). Cells were twice washed in ice cold homogenisation buffer containing 10 mMTris-HCl, 1 mM EDTA.2Na, 250 mM sucrose, 1 mM PMSF, 0.3 mM indomethacin, pH 7.4, homogenised and re-centrifuged as before. The supernatant was stored on ice and pellets re-homogenised and re-spun. Supernatants were pooled and centrifuged at 40000g, 10 minutes, 4°C. Resultant membrane pellets were stored at -80°C until use.

For assay, membranes expressing human $EP_4$, $EP_3$, $EP_2$ or $EP_1$ receptors were incubated in Millipore (MHVBN45) plates containing assay buffer, radiolabelled $[^3H]PGE_2$ and 0.1 to 10 000 nM concentrations of compounds. Incubations were performed at suitable temperatures and for suitable times to allow equilibrium to be reached. Non-specific binding was determined in the presence of 10uM $PGE_2$. Bound and free radiolabel was separated by vacuum manifold filtration using appropriate wash buffers, and bound radiolabel was determined by scintillation counting. Constituents of each of the buffers are included in table 4 below.

The affinity or $pK_i$ of each compound for each receptor was calculated from the concentration causing 50% radioligand displacement ($IC_{50}$) using the Cheng-Prusoff equation:

$$Ki = \frac{IC_{50}}{1 + \left( \dfrac{radioligand\ concentration}{radioligand\ KD} \right)}$$

This approach follows that set out in Kenakin, T.P., Pharmacologic analysis of drug receptor interaction. Raven Press, New York, 2nd edition.

*Table 4*

| Receptor | | $EP_1$ | $EP_2$ | $EP_3$ | $EP_4$ |
|---|---|---|---|---|---|
| Protein / well | | 6.5μg | 8μg | 5μg | 5μg |
| Final [$^3H$-$PGE_2$] | | 3.6nM | 3nM | 2.5nM | 1nM |
| Buffer | Assay | 10mM MES pH6.0; 10mM $MgCl_2$; 1mM EDTA, 3uM Indomethacin | 10mM MES pH6.0; 10mM $MgCl_2$; 1mM EDTA | 10mM MES pH 6.0; 10mM MgCl2; 1mm EDTA, 100uM GTP-gamma-S | 10mM MES pH6.0; 10mM $MgCl_2$; 1mM EDTA, 3uM Indomethacin |
| | Wash | 10mM MES pH6.0; 10mM $MgCl_2$ | 10mM MES pH6.0; 10mM $MgCl_2$ | 10mM MES pH 6.0; 10mM $MgCl_2$ | 10mM MES pH6.0; 1mM EDTA |

*Effect of compounds on cyclase production*

The following describes an in vitro assay to determine the effect of compounds on cyclase production, that is, to determine their functional efficacy at the $EP_2$ receptor.

Cell stimulation

HEK cells stably expressing the human EP2 receptor were used for these assays. HEK-EP2 cells were cultured in 96-well, poly-L-lysine coated plates at a density of 50,000 cells/well, and grown to confluence in humidified 95%$O_2$/5%$CO_2$ at 37˚C. Culture medium was DMEM supplemented with 10% foetal bovine serum, 100U/ml penicillin, 100ng/ml streptomycin, 2.5μg/ml fungizone, 2mM glutamine, 250μg/ml geneticin and 200μg/ml zeocin.

On reaching confluence, culture media was rinsed off using DMEM with no additions, before 175μl assay buffer (DMEM containing 1mM 3-isobutyl -1-methylxanthine and 3μM indomethacin) was added to each well. This was allowed to incubate for 1hr before the cells were stimulated with the test compounds (in triplicate) at final concentrations of $10^{-9}$M to $10^{-5}$M for 15 minutes. The assay was terminated by the addition of 25μl 1M hydrochloric acid. Plates were then frozen for a minimum of 12 hours or until required for radioligand displacement assay.

Radioligand displacement assay

Plates were thawed quickly at 37˚C, and neutralised with 25μl 1M sodium hydroxide. 30μl of supernatant was transferred to 96-well Millipore (MAFNOB) plates coated with 0.1% Polyethylenimine. These supernatants were diluted by addition of 90μl cAMP assay buffer (50mM Tris, 5mM EDTA, pH 7.0). A cAMP standard curve ($10^{-11}$M to $10^{-5}$M) was constructed. 15μl of 3':5'-cAMP-dependent protein kinase (final concentration 8μg/well), and 15μl [$^3H$]-cAMP (final concentration 2nM/well) were added to each well.

Plates were incubated on ice for 2 hours, before bound and free radiolabel were separated by vacuum filtration harvesting on the Millipore manifold, using ice cold water as the termination buffer. Filter plates were allowed to dry overnight, before addition of 50μl Microscint. Radioactivity was determined using the Microbeta Trilux scintillation counter. cAMP accumulation was determined from the standard curve, and the values plotted as pmoles cAMP/well.

*Effect of compounds on human myometrial activity*

The following describes an *in vitro* functional assay, using human myometrial smooth muscle, to determine the affinity of the test compounds at the $EP_2$ receptor in human tissues.

Sections of human myometrium were prepared from samples of surgically removed uterus longitudinal myometrial muscle strips (2mm wide by 10mm long) were then cut and suspended between stainless steel hooks in organ chambers containing oxygenated (95% $O_2$/5% $CO_2$) Krebs solution at 37˚C. The composition of the Krebs solution was as follows: NaCl (118.2mM) , KCl (4.69mM), $MgSO_4.7H_2O$ (1.18mM), $KH_2PO_4$ (1.19mM), glucose (11.1mM), $NaHCO_3$ (25.0mM), $CaCl_2.6H_2O$ (2.5mM), indomethacin 3x$10^{-6}$M.

Tissues were placed under a tension equivalent to 25mN and left overnight at room temperature. The following day the tissues were maintained at 37˚C, washed and placed under a tension of 15mN then allowed to equilibrate for a period of at least 30 minutes. Responses were recorded using isometric transducers coupled to an Apple Macintosh computer via a MacLab interface. After 60 minutes, the muscle sections of the human myometrium were stimulated electrically (15ms pulse width, for 10s every 100s at 15V and 0.5-40Hz) using parallel platinum wire electrodes and a

Multistim D330 pulse stimulator. Upon electrical stimulation, the strips of human myometrial smooth muscle responded with a rapid contraction. Once the response to electrical stimulation had stabilised (stimulated responses differed by no more than 10%), the strips were exposed to increasing concentrations of test compounds ($1x10^{-7}$ to $1x10^{-4}$M, incubated for at least 15 minutes at each concentration). At the end of the experiment, application of sodium nitroprusside (SNP, a nitric oxide donor that causes smooth muscle relaxation) ($1x10^{-4}$M) was used to produce a standard relaxatory response. To determine the affinity of the compounds, the concentration of test compound required to produce half-maximal effects ($EC_{50}$) was calculated, as was the maximum response (calculated as a percentage of the standard response produced with SNP).

## Results

*Binding ability to human EP receptors*

In these tests, the affinity of the four separated stereoisomers of AH-13205 were determined, and the results are shown in figure 4 (data is shown as mean±s.e for 4 experiments). The stereoisomer isolated in peak 1 of mixture 1 was shown to be the most potent, having a pKi of 7.1.

The full results of the binding tests are shown in table 5 as pKi values:

*Table 5*

| Compound | $EP_2$ | $EP_1$ | $EP_3$ | $EP_4$ |
|---|---|---|---|---|
| Peak 1, Mixture 1 acid | 7.1 | - | 5.7 | 5.0 |
| Peak 2, Mixture 1 acid | 5.8 | - | 4.8 | 4.5 |
| Peak 1, Mixture 2 acid | 6.9 | - | 4.8 | 5.1 |
| Peak 2, Mixture 2 acid | 6.3 | - | 4.7 | 4.8 |
| AH-13205 | 6.4 | 5.0 | 5.2 | 4.6 |

From this table, it can be seen that Peak 1, mixture 2 acid is the most selective of the stereoisomers.

*Effect of compounds on cAMP production*

In these tests, the effect of the separated stereoisomers and AH-13205 on cAMP production mediated by human EP receptor stimulation was assessed. All the compounds showed the same maximal response, but their potency differed, as shown in Figure 5 and table 6 (data is shown as mean±s.e. for 4 experiments).

*Table 6*

| Compound | Mean Log ($EC_{50}$) | S.E.M. | Mean EC50 (nM) |
|---|---|---|---|
| Peak 1, Mixture 1 acid | -8.01 | 0.22 | 10 |
| Peak 2, Mixture 1 acid | -6.49 | 0.19 | 323 |
| Peak 1, Mixture 2 acid | -7.25 | 0.19 | 56 |
| Peak 2, Mixture 2 acid | -6.39 | 0.24 | 407 |
| AH13205 | -7.49 | 0.29 | 32 |

*Effect of compounds on human myometrial activity*

Application of AH-13205 was shown to inhibit electrically-induced contractions in human myometrium - points A, B and C correspond to the addition of increasing amounts of AH-13205 ($10^{-6}$, $10^{-5}$ and $10^{-4}$ M) (Figure 6). The potency of the effect was in accordance with interaction at a prostaglandin $EP_2$ receptor, as the vehicle containing AH-13205 was shown to have no effect (Figure 7).

The effects of the two most potent stereoisomers (Peak 1, Mixture 1 acid and Peak 1, Mixture 2 acid) were investigated, and compared to the effects of AH-13205. Peak 1, Mixture 1 acid, Peak 1, Mixture 2 acid and AH13205, all caused concentration-dependent inhibition of the EFS-evoked response. The pEC50s were 5.9±0.2 (n=7), 5.3±0.1 (n=6) and 5.3±0.2 (n=7) (Figure 8). There was no significant differences between the maximum inhibitory effects observed, with inhibition of EFS-induced contractions of 56±5% (Peak 1, Mixture 1 acid), 57+2% (Peak 1, Mixture 2 acid) and 49±5% (AH-13205). SNP caused further inhibition on top of the compounds, equivalent to 60-70% of the control EFS response. The SNP inhibitory effect was reversed over a 60-80 minutes washing period but the inhibitory effects of the compounds tested were not.

In addition, the effect of terbutaline, a β adrenoceptor agonist, on EFS-induced contractions of myometrium was investigated, and shown to have no significant inhibitory effect on the EFS-evoked contractions (98±5% of the control EFS-induced contraction at $10^{-4}$M, n=7 donors).

**Example 7: Inhibition of IL-2, TNFα and IFN-γ production**

Lymphocytes are mononuclear leukocytes, which participate in specific immune responses to foreign antigens and in the manifestation of auto-immune diseases. T lymphocytes produce IL-2, a key factor for lymphocyte activation and proliferation, in response to antigen stimulation via the CD3-T cell receptor complex and the pathway involved in this response is the NF-AT. This response can be demonstrated in vitro by using selective monoclonal antibodies with specificity to the CD3 molecules on T cells. A lymphocyte assay was designed to model this response and to determine the effect of test compound on IL-2 production by anti-CD3-stimulated T cells isolated from peripheral blood. This assay uses a sub-optimal dose of an anti-CD3 monoclonal antibody (OKT3, 25ng/ml) immobilised to a 96-well plate to stimulate a T cell response. The level of IL-2 released into the cell culture supernatants was quantified using a standard sandwich ELISA. Similarly, other cytokines such as TNFα and IFN-γ can also be measured in the same assay. The assay can be extended to 72-hour time point when lymphocyte proliferation in response to anti-CD3 antibody can be observed, hence the effect of immune modulatory compounds examined.

Monocytes are peripheral mononuclear phagocytes that participate in inflammatory responses. TNFα production by monocytes plays an important role in inflammatory responses and can cause considerable tissue damage if the level remained unchecked. Inhibition of TNFα secretion by activated monocytes may provide an attractive therapy for the treatment of inflammatory conditions.

One of the most potent microbial triggers of TNFα release by monocytes is lipopolysaccharide (LPS) and this response is via the NF-KB pathway. A 96 well in vitro assay was established to determine the effects of test compounds on LPS-induced TNFα secretion by human peripheral blood monocytes. The level of TNFα in assay supernatants was quantified using a standard sandwich ELISA.

**Test Details**

Human peripheral blood mononuclear cells from healthy volunteers were isolated from whole blood by Ficoll-Hypaque density centrifugation and adherence to plastic. The non-adherent lymphocyte fraction was used to set up the lymphocyte assay and the adherent monocytes were then recovered by scraping and subsequently used in the monocyte assay.

*Lymphocyte assay*

Lymphocytes were then seeded to a 96-well plate pre-coated with anti-CD3 monoclonal antibody (OKT3) at 25 ng/ml and immediately, the test compounds (Peak 1, mixture 1 acid; Peak 1, mixture 2 acid; AH-13205 racemate; $PGE_2$) in appropriate dilutions were added to corresponding wells according to the experimental design. The plate was incubated for 24 hours at 37˚C with 5% $CO_2$ in air and supernatants were recovered for ELISA analysis at the end of incubation period. The levels of IFN-γ was assessed by using the ProteoPlex 16 well human cytokine array assay kit according to the manufacturer's instruction.

For the measurement of lymphocyte proliferation driven by immobilised anti-CD3 monoclonal antibody, the assay was set up in the same way as for the measurement of IL-2 release, except that the cells were cultured for 72 hours in the presence or absence of test compounds. Four hours prior to the termination of the proliferation assay, a novel tetrazolium compound solution supplied by Promega in the format of Cell Proliferation Assay Kit was added to individual wells according to the manufacturer's instruction. The plate was then placed back in the incubator for the remaining 4 hours and the colorimetric reaction was measured using a spectrophotometer at an absorbent wavelength of 490 nm (SpectraMax, Molecular Devices) according to the manufacturer's instruction.

*Monocyte assay*

For the monocyte assay, the cells were plated onto 96-well plates and pre-treated for 1 hour at 37˚C / 5%$CO_2$ with the test compound (Peak 1, mixture 1 acid; Peak 1, mixture 2 acid; AH-13205 racemate), followed by the addition of LPS (100ng/ml) to initiate the reaction. The plate was incubated for 24 hours and supernatants were recovered for

the measurement of TNFα production by ELISA.

*Macrophage assay*

Human lung parenchyma was cut into small pieces and perfused with ice-cold phosphate buffered saline (PBS) to remove contaminating blood and mucus. The tissues were then chopped with scissors in the presence of Minimum Essential Medium supplemented with penicillin, streptomycin, L-glutamine and DNase (0.25 mg/ml). The chopped tissues were shaken gently to dislodge the macrophages. A crude cell suspension was then obtained by the removal of the tissues with a sterile filter (150 μm pore size). The resulting cell suspension was spun and the cell pellet collected. Contaminating red blood cells were depleted with a red blood cell lysis buffer and the remaining cells washed twice with PBS by centrifugation. Alveolar macrophages were then purified from this cell preparation by using a positive selection method for CDl4-molecule bearing cells using a VarioMac™ Separator and respective positive selection reagents and columns supplied by Miltenyi Biotec Ltd according to the manufacturer's instruction.

For the assay, alveolar macrophages were resuspended in complete culture medium consisting of RPMI1640 supplemented with 10% foetal calf serum, L-glutamine and antibiotics. The cells were then plated into 24 well plates ($4x10^5$ cell/well) and incubated overnight at 37°C with 5% $CO_2$ in air to allow cell adherence. The exhausted medium was then removed from the plates and the plates rinsed briefly with fresh medium before the addition of test compound solutions. The test compounds were incubated with the cells for 30 minutes before the addition of *E. Coli* LPS (1 μg/ml). The assay plates were incubated in a humidified incubator at 37°C with 5% $CO_2$ in air for 24 hours. The release of $TNF_\alpha$ by the cells into the culture supernatants was quantified using an ELISA kit (DuoSet® human $TNF_\alpha$ ELSIA Development System) supplied by R + D Systems (Europe) according to the manufacturer's instruction. Indomethacin at 3 μM was included in all treatments to inhibit the possible release of endogenous prostaglandin $E_2$.

**Results**

*Lymphocyte assay*

Figure 9 shows the results of IL-2 production by three test compounds given as mean of four donors (except peak 1, mixture 2 acid which was tested in one donor only). These results are summarized in table 7.

*Table 7*

| Compound | Mean Log ($EC_{50}$) | Mean EC50 (μM) |
|---|---|---|
| Peak 1, Mixture 1 acid | -6.006 | 0.986 |
| AH13205, racemate | -5.549 | 2.823 |
| $PGE_2$ | -7.554 | 0.028 |

These results show that $EP_2$ agonists concentration-dependently inhibit IL-2 production by OKT3 activated T cells. The order of potencies in the assay is $PGE_2$ > Peak 1, Mixture 1 acid > AH13205 (racemate) according to their respective $EC_{50}$ values.

Figure 10 shows the results given as mean of three to five donors of IL-2 production by three EP2 receptor agonists. These results are summarized in table 8.

*Table 8*

| Compound | Mean Log ($EC_{50}$) | Mean EC50 (μM) |
|---|---|---|
| Peak 1, Mixture 1 acid | -5.880 | 1.319 |
| Butaprost | -6.906 | 0.1242 |
| $PGE_2$ | -7.677 | 0.02105 |

These results show that $EP_2$ agonists concentration-dependently inhibit IL-2 production by OKT3 activated T cells. The order of potencies in the assay is $PGE_2$ > butaprost> Peak 1, Mixture 1 acid according to their respective $EC_{50}$ values.

Figure 11 shows the results from two donors of interferon gamma release by Peak 1, Mixture 1 acid. These results show that $EP_2$ agonists concentration-dependently inhibit interferon gamma release.

Figure 12 shows the results given as mean of three donors of TNFα production by three EP2 receptor agonists.

These results are summarized in table 9.

*Table 9*

| Compound | Mean Log ($EC_{50}$) | Mean EC50 (μM) |
|---|---|---|
| Peak 1, Mixture 1 acid | -5.632 | 2.332 |
| Butaprost | -6.535 | 0.2917 |
| $PGE_2$ | -7.971 | 0.01069 |

These results show that $EP_2$ agonists concentration-dependently inhibit TNFα production by lymphocytes. The order of potencies in the assay is $PGE_2$ > butaprost> Peak 1, Mixture 1 acid according to their respective $EC_{50}$ values.

Figure 13 shows the results given as mean of three donors of lymphocyte proliferation by three $EP_2$ receptor agonists. These results are summarized in table 10.

*Table 10*

| Compound | Mean Log ($EC_{50}$) | Mean EC50 (μM) |
|---|---|---|
| Peak 1, Mixture 1 acid | -5.000 | 9.995 |
| Butaprost | -6.015 | 0.996 |
| $PGE_2$ | -7.589 | 0.02579 |

These results show that $EP_2$ agonists concentration-dependently inhibit lymphocyte proliferation. The order of potencies in the assay is $PGE_2$ > butaprost> Peak 1, Mixture 1 acid according to their respective $EC_{50}$ values.

*Monocyte assay*

Figure 14 shows the results given as mean of three donors. These results are summarized in table 11.

*Table 11*

| Compound | Mean Log ($EC_{50}$) | Mean EC50 (μM) |
|---|---|---|
| Peak 1, Mixture 1 acid | -5.749 | 1.783 |
| Peak 1, Mixture 2 acid | -5.172 | 6.730 |
| AH13205, racemate | -4.704 | 19.780 |

These results show that the $EP_2$ agonists concentration-dependently inhibited TNFα production by LPS-stimulated monocytes. The order of potencies based on their respective $EC_{50}$ values is Peak 1, Mixture 1 acid > Peak 1, Mixture 2 acid > AH13205 (racemate).

*Macrophage assay*

Figure 15 shows the results given as mean of three donors. These results are summarized in table 12.

*Table 12*

| Compound | Mean Log ($EC_{50}$) | Mean EC50 (μM) |
|---|---|---|
| Peak 1, Mixture 1 acid | -5.154 | 7.023 |
| $PGE_2$ | -6.904 | 0.1247 |

These results show that the $EP_2$ agonists concentration-dependently inhibited TNFα production by macrophages. The order of potencies based on their respective $EC_{50}$ values is $PGE_2$ > Peak 1, Mixture 1 acid.

**Key to Figures**

*Figures 2a and 2b*

| | |
|---|---|
| ———————— | R-BPH |
| - - - | S-BPH |

*Figures 3a and 3b*

| | |
|---|---|
| - | Peak 1, mixture 1 acid |
| - - | Peak 2, mixture 1 acid |
| - - - | Peak 1, mixture 2 acid |
| - - - - - - | Peak 2, mixture 2 acid |

*Figure 4*

| | |
|---|---|
| ■ | Peak 1, mixture 1 acid |
| ▲ | Peak 2, mixture 1 acid |
| □ | Peak 1, mixture 2 acid |
| ○ | Peak 2, mixture 2 acid |
| • | AH-13205 (racemate) |

*Figure 5*

| | |
|---|---|
| ■ | Peak 1, mixture 1 acid |
| ▲ | Peak 2, mixture 1 acid |
| • | Peak 1, mixture 2 acid |
| ○ | Peak 2, mixture 2 acid |
| □ | AH-13205 (racemate) |

*Figure 7*

| | |
|---|---|
| □ | Vehicle alone |
| ■ | Vehicle + AH13205 |

*Figure 8*

| | |
|---|---|
| □ | Peak 1, mixture 1 acid |
| ■ | Peak 1, mixture 2 acid |
| O | AH-13205 (racemate) |

*Figure 9*

| | |
|---|---|
| ■ | Peak 1, mixture 1 acid |
| □ | Peak 1, mixture 2 acid |
| ○ | AH-13205 (racemate) |
| ▲ | $PGE_2$ |

*Figure 10*

| | |
|---|---|
| ● | Peak 1, mixture 1 |
| ▲ | butaprost |
| ○ | $PGE_2$ |

*Figure 11*

| | |
|---|---|
| ■ | Donor 1 |
| □ | Donor 2 |

*Figure 12*

| | |
|---|---|
| ▼ | Peak 1, mixture 1 |
| ▲ | butaprost |
| ■ | $PGE_2$ |

*Figure 13*

| | |
|---|---|
| ▼ | Peak 1, mixture 1 |
| ▲ | butaprost |
| ■ | $PGE_2$ |

*Figure 14*

| | |
|---|---|
| ■ | Peak 1, mixture 1 |
| □ | Peak 1, mixture 2 |
| ● | AH-13205 (racemate) |

*Figure 15*

| | |
|---|---|
| ▲ | Peak 1, mixture 1 |
| ■ | $PGE_2$ |

## Claims

**1.** A compound selected from one of the following:

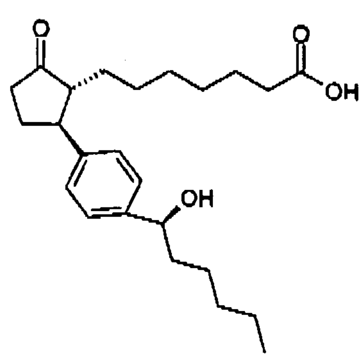

(1R,2S)-2-[4-(1-(S)-hydroxyhexyl)phenyl]-5-oxo-cyclopentaneheptanoic acid [RSS] ; or

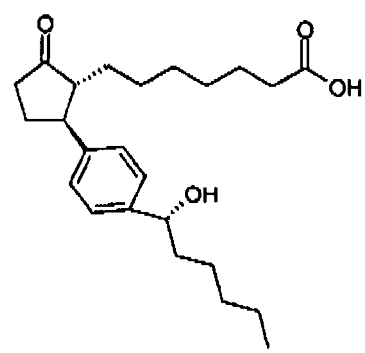

(1R,2S)-2-[4-(1-(R)-hydroxyhexyl)phenyl]-5-oxo-cyclopentaneheptanoic acid [RSR] ,

or a salt or solvate thereof.

**2.** (*trans*-2-[4-(1-hydroxyhexyl)phenyl]-5-oxo-cyclopentaneheptanoic acid, of which at least 90% by weight is selected from one of the following forms:

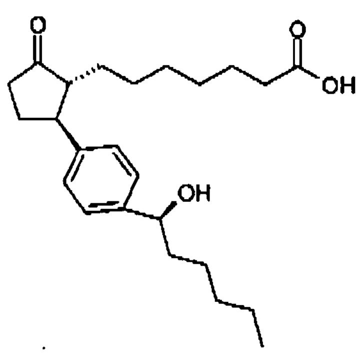

(1R,2S)-2-[4-(1-(S)-hydroxyhexyl)phenyl]-
5-oxo-cyclopentaneheptanoic acid
[RSS] ; or

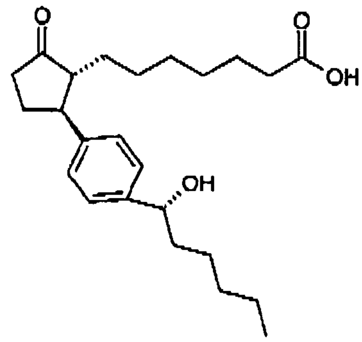

(1R,2S)-2-[4-(1-(R)-hydroxyhexyl)phenyl]-
5-oxo-cyclopentaneheptanoic acid
[RSR] ,

or a salt or solvate thereof.

**3.** 2-[4-(1-hydroxyhexyl)phenyl]-5-oxo-cyclopentaneheptanoic acid, of which at least 80% by weight is in one of the following forms:

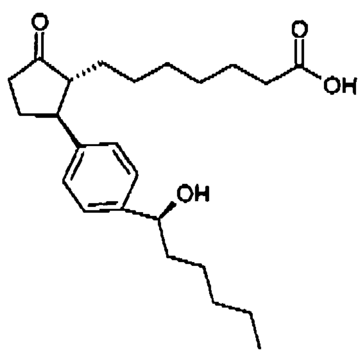

(1R,2S)-2-[4-(1-(S)-hydroxyhexyl)phenyl]-
5-oxo-cyclopentaneheptanoic acid
[RSS] ; or

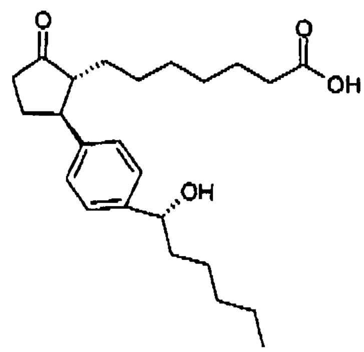

(1R,2S)-2-[4-(1-(R)-hydroxyhexyl)phenyl]-
5-oxo-cyclopentaneheptanoic acid
[RSR] ,

or a salt or solvate thereof.

**4.** A compound according to any one of claims 1 to 3, or a pharmaceutically acceptable salt thereof, for use in a method of therapy.

**5.** A pharmaceutical composition comprising a compound according to any one of claims 1 to 3, or a pharmaceutically acceptable salt thereof, together with a pharmaceutically acceptable carrier or diluent.

**6.** The use of a compound according to any one of claims 1 to 3, or a pharmaceutically acceptable salt thereof in the preparation of a medicament for the treatment of a condition alleviated by agonism of an $EP_2$ receptor.

**7.** The use according to claim 7, wherein the condition alleviated by agonism of an $EP_2$ receptor is selected from the group consisting of: glaucoma, dysmenorrhoea and pre-term labour.

**8.** The use of an $EP_2$ receptor agonist according to any one of claims 1 to 3, or a pharmaceutically acceptable salt thereof in the preparation of a medicament for the treatment of a condition alleviated by the inhibition of:

(i) human T-cell activation (proliferation);

(ii) the release of IL-2; or
(iii) the release of IFNγ.

**9.** The use according to claim 9, wherein the condition is a condition alleviated by the inhibition of:

(ii) the release of IL-2; or.
(iii) the release of IFNγ.

**10.** The use of an $EP_2$ receptor agonist according to any one of claims 1 to 3, or a pharmaceutically acceptable salt thereof in the preparation of a medicament for the treatment of psoriasis.

**11.** A compound according to any one of claims 1 to 3, or a pharmaceutically acceptable salt thereof, for use in a method of treatment of a condition alleviated by agonism of an $EP_2$ receptor.

**Patentansprüche**

**1.** Verbindung, die aus einer der folgenden ausgewählt ist:

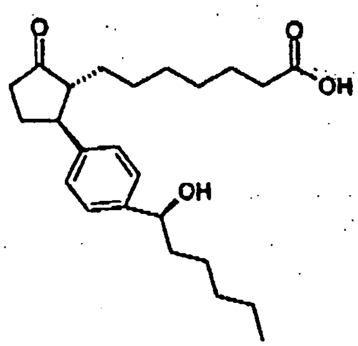

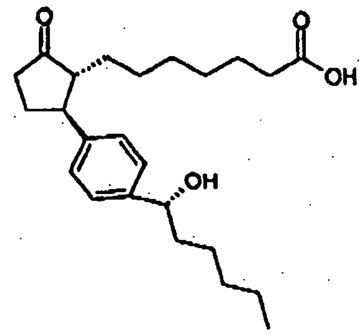

oder

(1R,2S)-2-[4-(1-(S)-Hydroxyhexyl)phenyl]-5-oxo-cyclopentanheptansäure [RSS];

(1R,2S)-2-[4-(1-(R)-Hydroxyhexyl)-phenyl]-5-oxocyclopentanheptan-säure [RSR],

oder ein Salz oder Solvat davon.

**2.** (trans-2-[4-(1-Hydroxyhexyl)phenyl]-5-oxocyclopentanheptansäure, von der zumindest 90 Gew.-% aus einer der folgenden Formen ausgewählt sind:

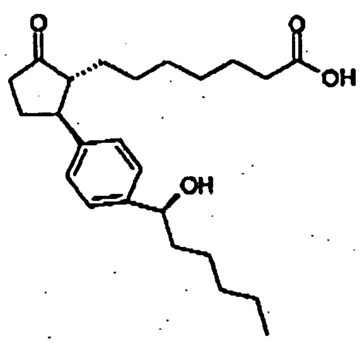

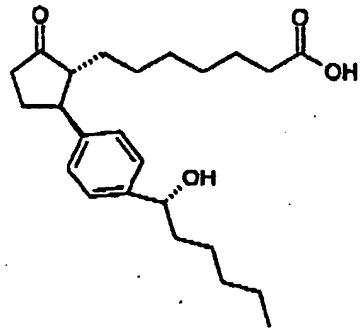

oder

(1R,2S)-2-[4-(1-(S)-Hydroxyhexyl)phenyl]-5-oxo-cyclopentanheptansäure [RSS]; (1R,2S)-2-[4-(1-(R)-Hydroxyhexyl)-phenyl]-5-oxocyclopentanheptansäure [RSR],

oder ein Salz oder Solvat davon.

3. 2-[4-(1-Hydroxyhexyl)phenyl]-5-oxocyclopentanheptansäure, von der zumindest 80 Gew.-% in einer der folgenden Formen vorliegen:

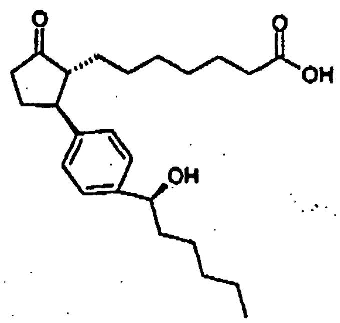

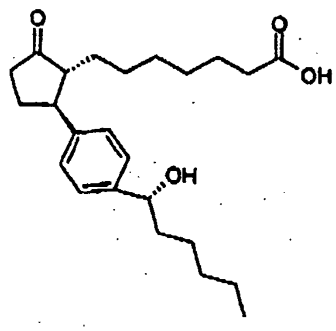

oder

(1R,2S)-2-[4-(1-(S)-Hydroxyhexyl)phenyl]-5-oxo-cyclopentanheptansäure [RSS]; (1R,2S)-2-[4-(1-(R)-Hydroxyhexyl)-phenyl]-5-oxocyclopentanheptansäure [RSR],

oder ein Salz oder Solvat davon.

4. Verbindung nach einem der Ansprüche 1 bis 3, oder ein pharmazeutisch annehmbares Salz davon, zur Verwendung in einem Therapieverfahren.

5. Pharmazeutische Zusammensetzung, umfassend eine Verbindung nach einem der Ansprüche 1 bis 3, oder ein pharmazeutisch annehmbares Salz davon, zusammen mit einem pharmazeutisch annehmbaren Träger oder Verdünnungsmittel.

6. Verwendung einer Verbindung nach einem der Ansprüche 1 bis 3, oder eines pharmazeutisch annehmbaren Salzes davon, zur Herstellung eines Medikaments zur Behandlung einer Erkrankung, die durch Agonismus eines $EP_2$-Rezeptors gelindert wird.

7. Verwendung nach Anspruch 6, worin die Erkrankung, die durch Agonismus eines $EP_2$-Rezeptors gelindert wird,

aus der aus Glaukom, Dysmenorrhoe und vorzeitigen Wehen bestehenden Gruppe ausgewählt ist.

**8.** Verwendung eines $EP_2$-Rezeptor-Agonisten nach einem der Ansprüche 1 bis 3, oder eines pharmazeutisch annehmbaren Salzes davon, zur Herstellung eines Medikaments zur Behandlung einer Erkrankung, die durch die Inhibierung von Folgendem gelindert wird:

(i) Aktivierung (Proliferation) menschlicher T-Zellen;
(ii) Freisetzung von IL-2; oder
(iii) Freisetzung von IFNγ.

**9.** Verwendung nach Anspruch 8, worin die Erkrankung eine Erkrankung ist, die durch die Inhibierung von Folgendem gelindert wird:

(ii) Freisetzung von IL-2; oder
(iii) Freisetzung von IFNγ.

**10.** Verwendung eines $EP_2$-Rezeptor-Agonisten nach einem der Ansprüche 1 bis 3, oder eines pharmazeutisch annehmbaren Salzes davon, zur Herstellung eines Medikaments zur Behandlung von Psoriasis.

**11.** Verbindung nach einem der Ansprüche 1 bis 3, oder ein pharmazeutisch annehmbares Salz davon, zur Verwendung in einem Behandlungsverfahren für eine Erkrankung, die durch Agonismus eines $EP_2$-Rezeptors gelindert wird.

**Revendications**

**1.** Composé sélectionné parmi un des suivants:

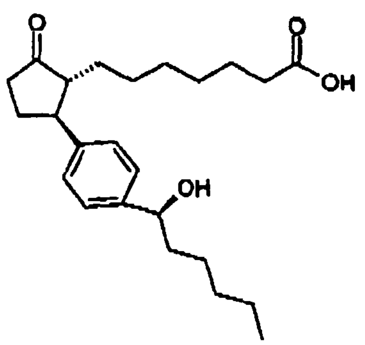

Acide (1R,2S)-2[4-(1-(S)-hydroxyhéxyl)phényl]-5-oxo-cyclopentaneheptanoïque) [RSS]

; ou

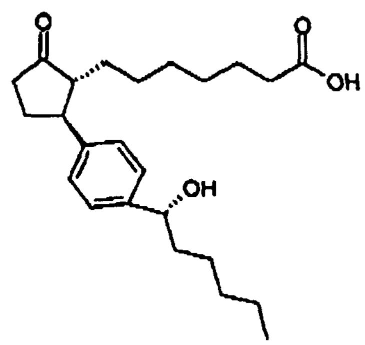

Acide (1R,2S)-2[4-(1-(R)-hydroxyhéxyl)phényl]-5-oxo-cyclopentaneheptanoïque [RSR]

ou un sel ou solvate de celui-ci.

**2.** Acide (*trans*-2-[4-(1-hydroxyphényl)phényl)-5-oxo-cyclopentaneheptanoique, dont au moins 90% en poids est sélectionné d'une des formes suivantes:

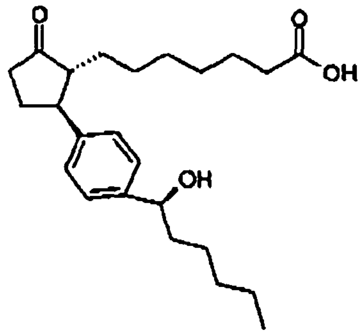

Acide (1R,2S)-2[4-(1-(S)-hydroxyhéxyl)phényl]-5-oxo-cyclopentaneheptanoïque) [RSS]

; ou

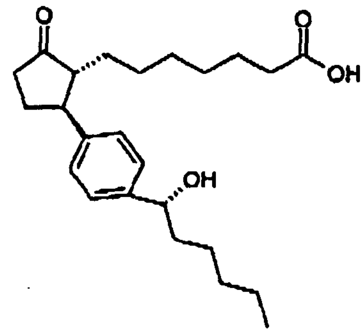

Acide (1R,2S)-2[4-(1-(R)-hydroxyhéxyl)phényl]-5-oxo-cyclopentaneheptanoïque [RSR]

ou un sel ou solvate de celui-ci.

**3.** Acide 2-[4-(1-hydroxyhéxyl)phényl)-5-oxo-cyclo-pentaneheptanoique, dont au moins 80% en poids se présente sous une des formes suivantes:

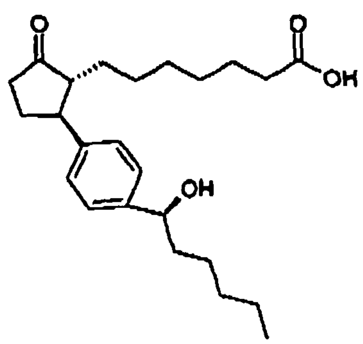

Acide (1R,2S)-2[4-(1-(S)-hydroxyhéxyl)phényl]-5-oxo-cyclopentaneheptanoïque) [RSS]

; ou

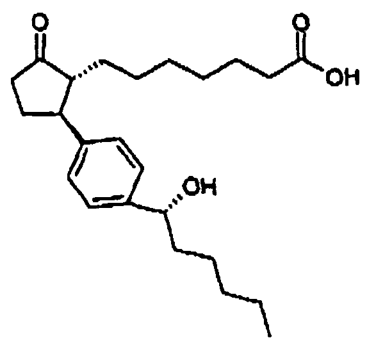

Acide (1R,2S)-2[4-(1-(R)-hydroxyhéxyl)phényl]-5-oxo-cyclopentaneheptanoïque [RSR]

ou un sel ou solvate de celui-ci.

**4.** Composé selon l'une quelconque des revendications 1 à 3, ou un sel pharmaceutiquement acceptable de celui-ci, pour utilisation dans une méthode de thérapie.

**5.** Composition pharmaceutique comprenant un composé selon l'une des revendications 1 à 3, ou un sel pharmaceutiquement acceptable de celui-ci, ensemble avec un support ou diluant pharmaceutiquement acceptable.

**6.** Utilisation d'un composé selon l'une quelconque des revendications 1 à 3, ou un sel pharmaceutiquement acceptable de celui-ci dans la préparation d'un médicament pour le traitement d'un état soulagé par l'agonisme d'un récepteur $EP_2$.

**7.** Utilisation selon la revendication 7, où l'état soulagé par l'agonisme d'un récepteur $EP_2$ est sélectionné dans le groupe constitué de: glaucome, dysménorrhée et accouchement avant terme.

**8.** Utilisation d'un agoniste du récepteur $EP_2$ selon l'une quelconque des revendications 1 à 3, ou un sel pharmaceutiquement acceptable de celui-ci dans la préparation d'un médicament pour le traitement d'un état soulagé par l'inhibition de:

(i) activation de lymphocytes T humains (prolifération),
(ii) la libération de IL-2; ou
(iii) la libération de IFNγ.

**9.** Utilisation selon la revendication 8, où l'état est un état soulagé par l'inhibition de:

(ii) la libération de IL-2; ou
(iii) la libération de IFNγ.

**10.** Utilisation d'un agoniste du récepteur $EP_2$ selon l'une quelconque des revendications 1 à 3, ou un sel pharmaceutiquement acceptable de celui-ci dans la préparation d'un médicament pour le traitement de psoriasis.

**11.** Composé selon l'une quelconque des revendications 1 à 3, ou un sel pharmaceutiquement acceptable de celui-ci, pour utilisation dans une méthode de traitement d'un état soulagé par l'agonisme d'un récepteur $EP_2$.

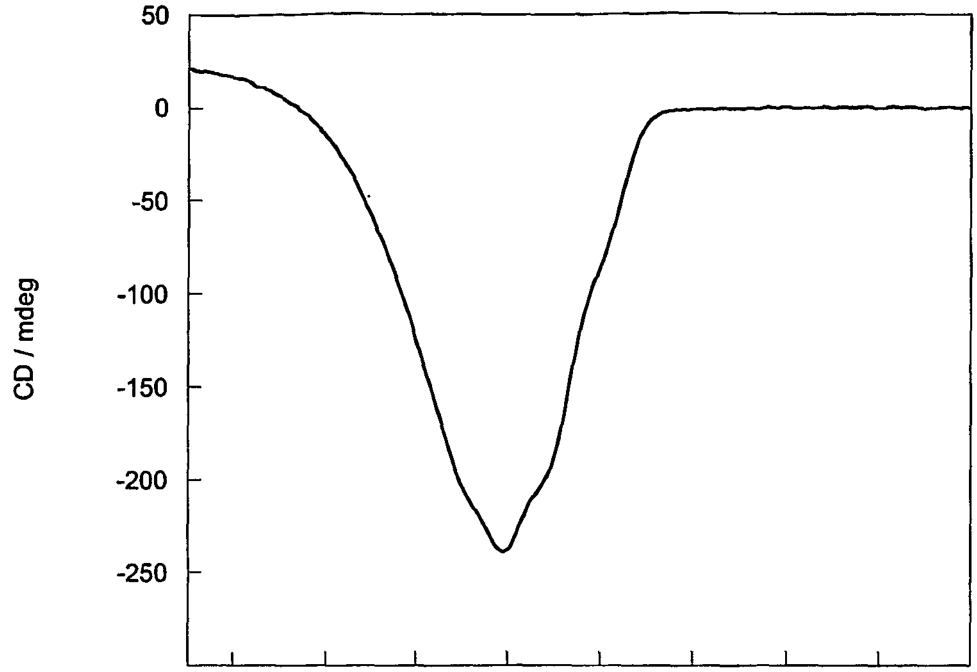
50
0
-50
-100
-150
-200
-250
CD / mdeg

Fig. 1a

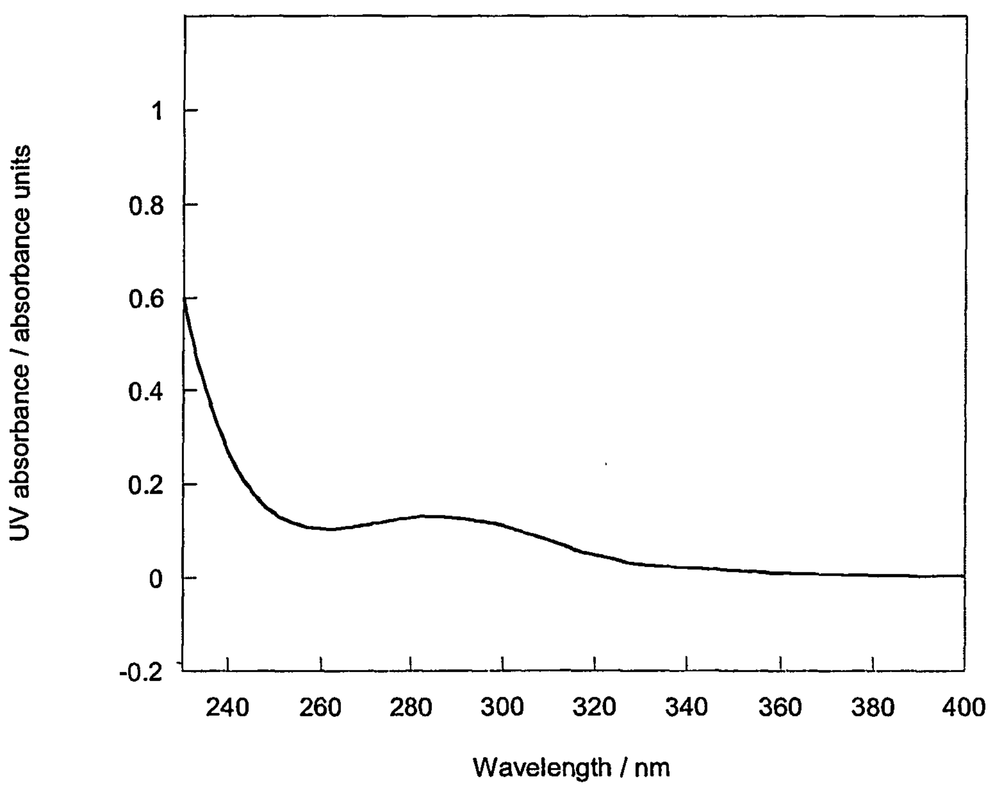
1
0.8
0.6
0.4
0.2
0
-0.2
UV absorbance / absorbance units
240
260
280
300
320
340
360
380
400
Wavelength / nm

Fig. 1b

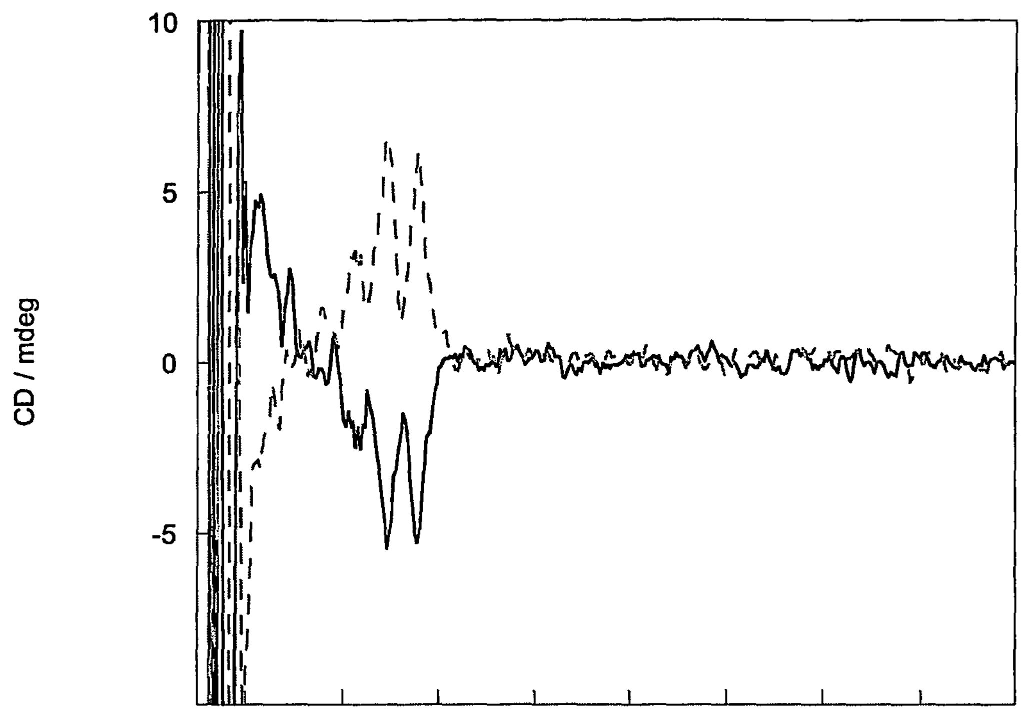
10
5
0
-5
CD / mdeg

Fig. 2a

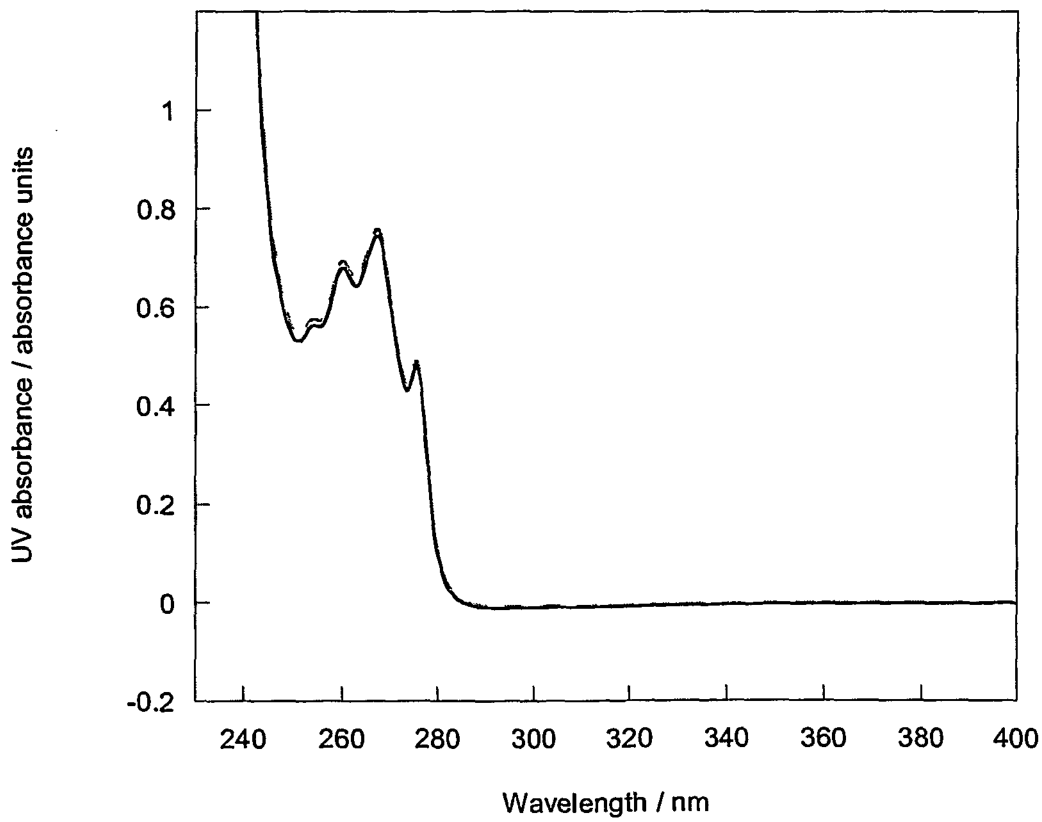
1
0.8
0.6
0.4
0.2
0
-0.2
UV absorbance / absorbance units
240
260
280
300
320
340
360
380
400
Wavelength / nm

Fig. 2b

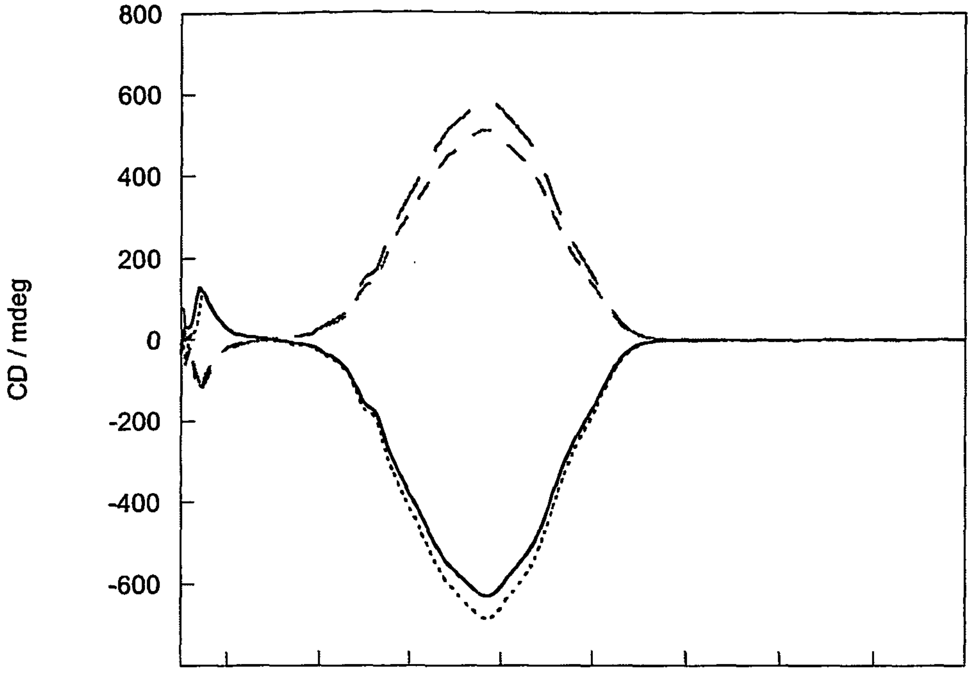
800
600
400
200
0
-200
-400
-600
CD / mdeg

Fig. 3a

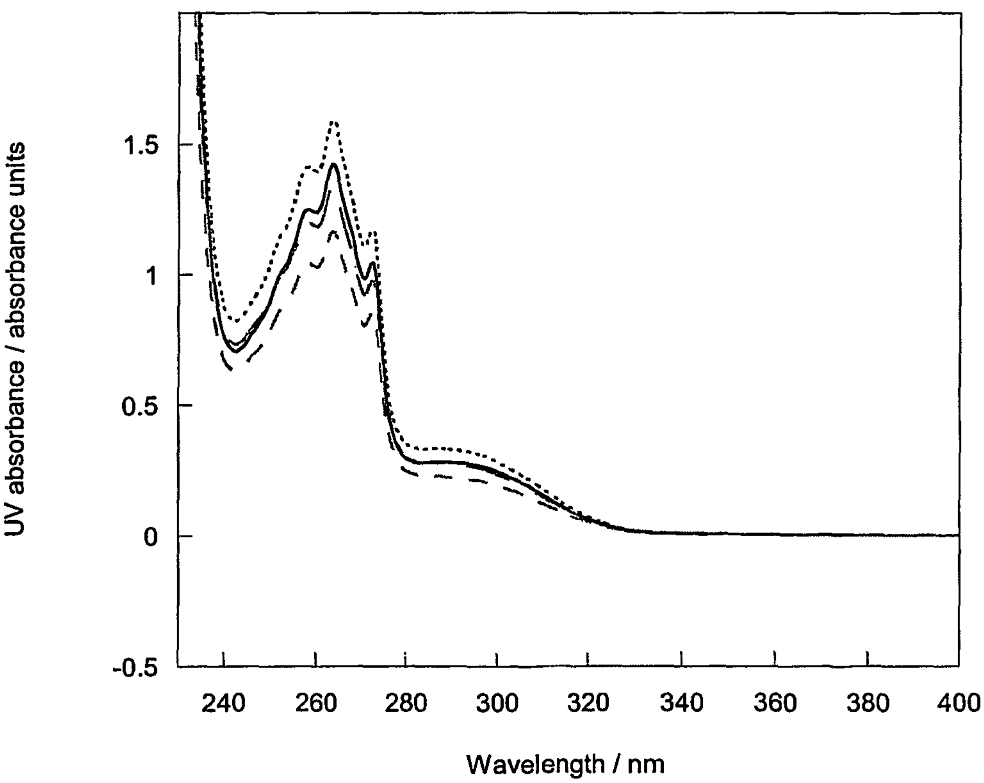
1.5
1
0.5
0
-0.5
UV absorbance / absorbance units
240
260
280
300
320
340
360
380
400
Wavelength / nm

Fig. 3b

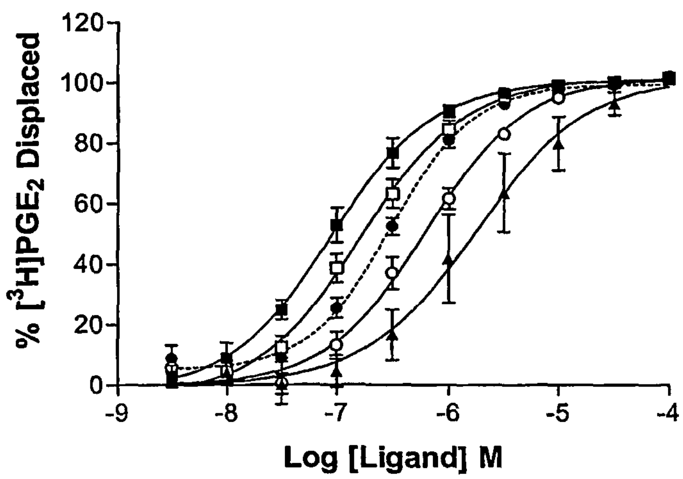
% [³H]PGE₂ Displaced
120
100
80
60
40
20
0
-9
-8
-7
-6
-5
-4
Log [Ligand] M

Fig. 4

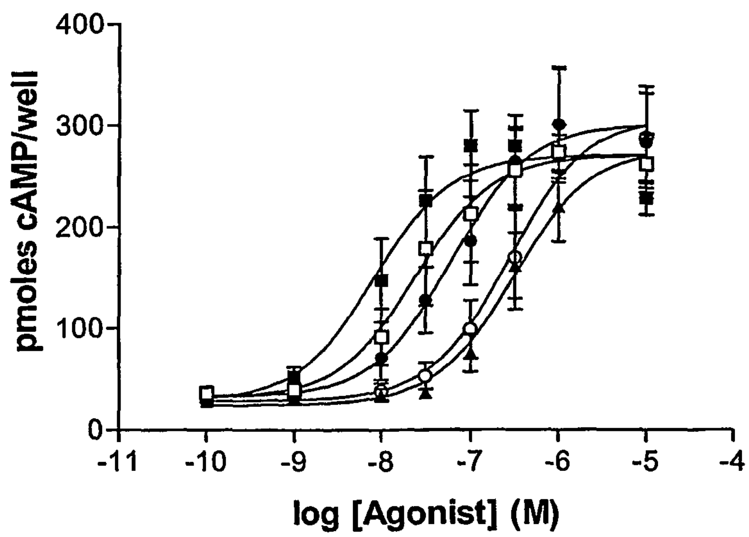
pmoles cAMP/well
400
300
200
100
0
-11
-10
-9
-8
-7
-6
-5
-4
log [Agonist] (M)

Fig. 5

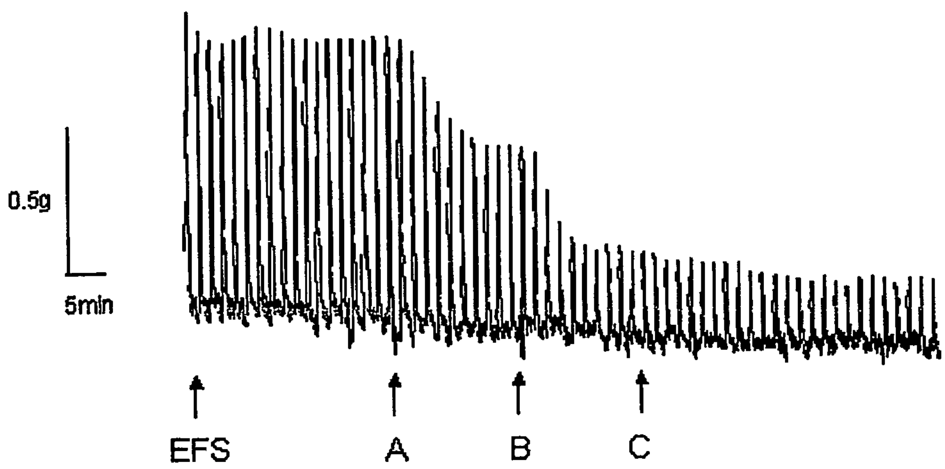
0.5g
5min
EFS
A
B
C

Fig. 6

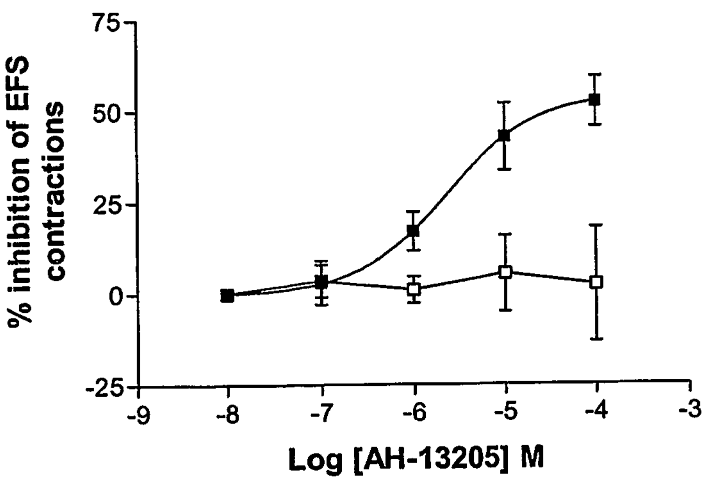
% inhibition of EFS contractions
75
50
25
0
-25
-9
-8
-7
-6
-5
-4
-3
Log [AH-13205] M

Fig. 7

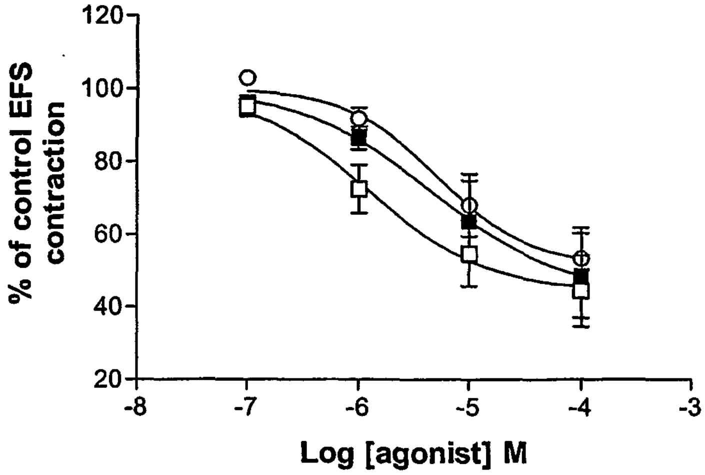
120
100
80
60
40
20
-8
-7
-6
-5
-4
-3
% of control EFS contraction
Log [agonist] M

Fig. 8

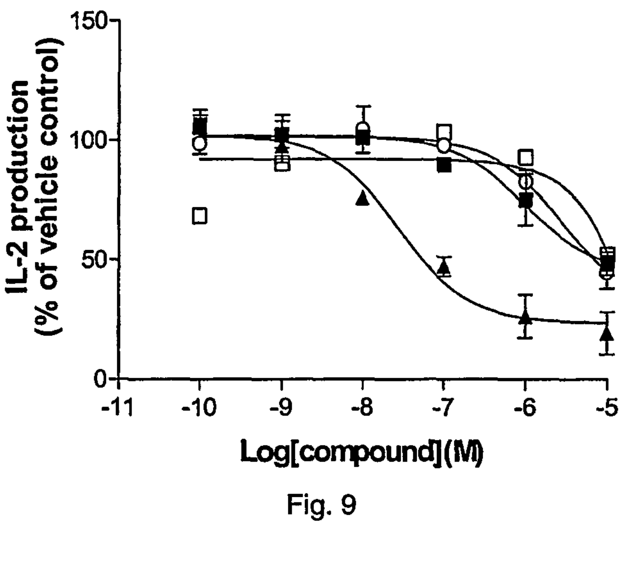
150
100
50
0
-11
-10
-9
-8
-7
-6
-5
IL-2 production (% of vehicle control)
Log[compound](M)

Fig. 9

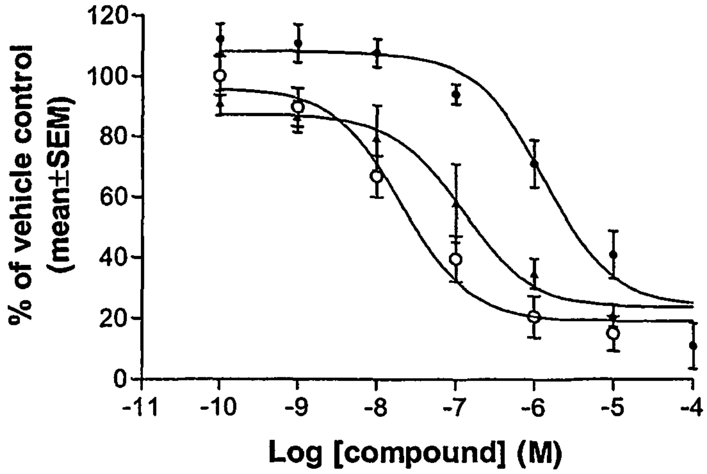
120
100
80
60
40
20
0
% of vehicle control (mean±SEM)
-11
-10
-9
-8
-7
-6
-5
-4
Log [compound] (M)

Fig. 10

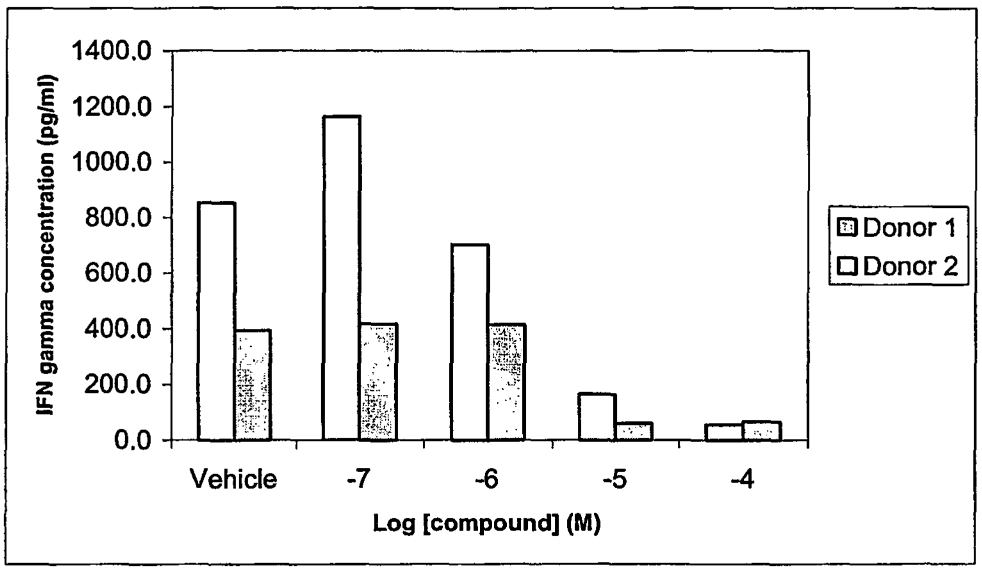
1400.0
1200.0
1000.0
800.0
600.0
400.0
200.0
0.0
IFN gamma concentration (pg/ml)
Donor 1
Donor 2
Vehicle
-7
-6
-5
-4
Log [compound] (M)

Fig. 11

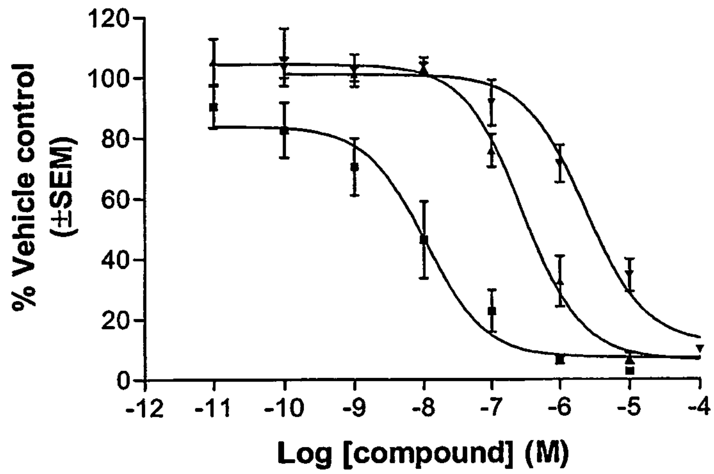
120
100
80
60
40
20
0
-12
-11
-10
-9
-8
-7
-6
-5
-4
% Vehicle control (±SEM)
Log [compound] (M)

Fig. 12

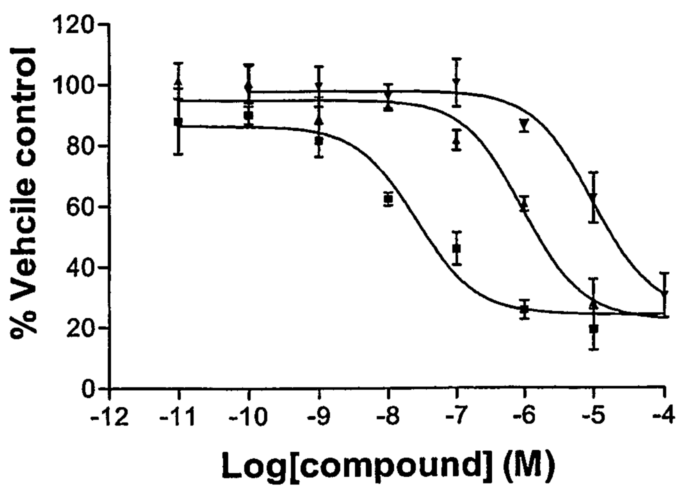
120
100
80
60
40
20
0
-12
-11
-10
-9
-8
-7
-6
-5
-4
% Vehcile control
Log[compound] (M)

Fig. 13

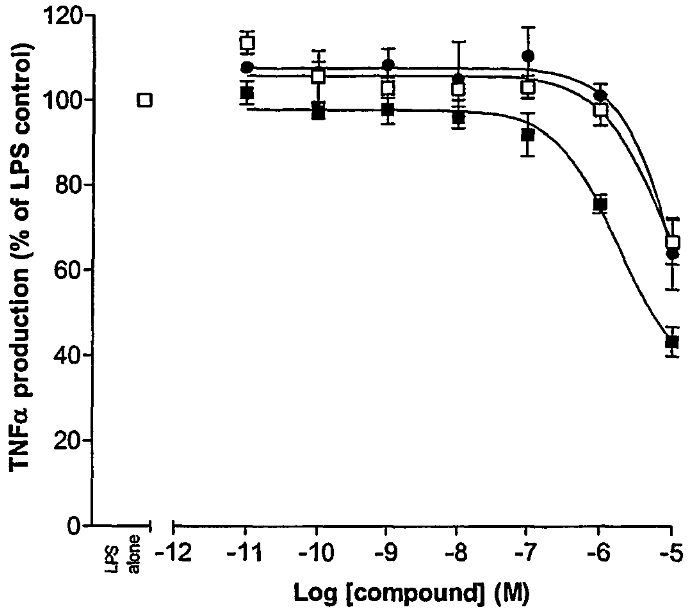
120
100
80
60
40
20
0
TNFα production (% of LPS control)
LPS alone
-12
-11
-10
-9
-8
-7
-6
-5
Log [compound] (M)

Fig. 14

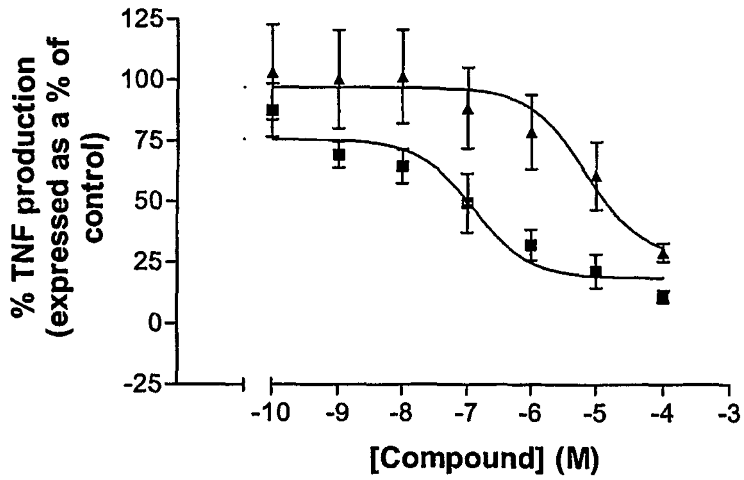
125
100
75
50
25
0
-25
% TNF production (expressed as a % of control)
-10
-9
-8
-7
-6
-5
-4
-3
[Compound] (M)

Fig. 15

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 03037433 A
- GB 2293101 A
- WO 03040126 A
- WO 9827976 A
- WO 0146140 A
- WO 0224647 A
- WO 0040248 A
- US 6562868 B
- WO 0031084 A
- US 5576347 A
- WO 9834916 A
- US 5889052 A
- US 4599353 A
- US 6011062 A
- US 6235781 B
- US 5849792 A
- US 5631287 A


### Non-patent literature cited in the description


- **COLEMAN, R.A.** Prostanoid Receptors. IUPHAR compendium of receptor characterisation and classification. 2000
- **BREYER, R.M. et al.** *Ann. N.Y. Acad. Sci.,* 2000, vol. 905, 221-231
- **HILLOCK, C.J. ; CRANKSHAW, D.J.** *European Journal of Pharmacology,* 1999, vol. 378, 99-108
- **GRIFFITHS, C.** *Current Drugs Targets - Inflammation & Allergy,* 2004, vol. 3, 157-161
- **LEBWOHL, M.** *Lancet,* 2003, vol. 361, 1197-1204
- **SALIM, A ; EMERSON, R.** *Curr. Open. Investig. Drug,* 2001, vol. 2 (11), 1546-8
- **BERGE et al.** *J. Pharm. Sci.,* 1977, vol. 66, 1-19
- **T. GREEN ; P. WUTS.** Protective Groups in Organic Synthesis. John Wiley and Sons, 1999
- Remington's Pharmaceutical Sciences. Lippincott, Williams & Wilkins, 2000
- **HAYASHI, T. et al.** *JACS,* 2002, vol. 124, 12102-12103
- **BROWN, H.C. et al.** *J. Am. Chem. Soc.,* 1988, vol. 110, 1539-1546
- **SUZUKI, M. et al.** *J. Am.Chem.Soc.,* 1985, vol. 107, 3348-3349
- **TABER, D.F. et al.** *J. Org. Chem.,* 1997, vol. 62, 194-198
- **PATTERSON, J.W. et al.** *Synthesis,* 1985, 337-338
- *Syn.Comms.,* 1989, 1509
- **WEILER, L.** *J. Am.Chem. Soc.,* 1970, vol. 92, 6702-6704
- Modern Synthetic Reactions. H.O. House, 1972, 553
- **HUCKIN, S.N. ; WEILER, L.** *Can. J. Chem.,* 1974, vol. 52, 2157
- Modern Synthetic Reactions. H.O. House, 1972, 511-517
- **TAKAYA, Y. et al.** *J. Am. Chem. Soc.,* 1998, vol. 120, 5579-5580
- **HAYASHI, T.** *Synlett, SI,* 2001, 879-887
- **THOMPSON, W.J. ; GAUDINO, J.** *J. Org. Chem.,* 1984, vol. 49, 5237-5243
- **FUNK, R.L. et al.** *J. Am. Chem. Soc,* 1993, vol. 115, 8849-8850
- *Tetrahedron,* 1996, vol. 52, 971-986
- **BROWN, H.C. et al.** *J. Am. Chem. Soc.,* 1998, vol. 110, 1539-1546
- **BAGLI, J. et al.** *J. Org. Chem.,* 1972, vol. 37, 2132-2138
- **BERNADY, K.F.** *J. Org. Chem.,* 1980, vol. 45, 4702-4715